# FASCICULE DE BREVET EUROPEEN

(11) **EP 0 378 953 B1**
(45) Date de publication et mention de la délivrance du brevet: **05.06.1996**
(21) Numéro de dépôt: 89420520.2
(22) Date de dépôt: 27.12.1989
(51) Int. Cl.: C07D 249/08, A01N 43/50, A01N 43/653, C07D 233/60, C07C 49/683, C07C 49/697, C07C 49/753, C07C 205/45, C07D 303/08, C07D 303/22, C07D 405/06

(54) **Benzylidène azolylméthylecycloalcane et utilisation comme fongicide**
Benzyliden-azolylmethylcycloalkan und Anwendung als Fungizid
Benzylidene azolyl methyl cycloalkane and its use as a fungicide

(30) Priorité: 29.12.1988 FR 8817580; 30.06.1989 FR 8909079; 13.07.1989 FR 8909741
(43) Date de publication de la demande: 25.07.1990
(73) Titulaire: RHONE-POULENC AGROCHIMIE, 69009 Lyon (FR)
(72) Inventeur: Hutt, Jeann, F-69009 Lyon (FR); Mugnier, Jacques, F-74330 La Balme de Lillingy (FR); Pepin, Régis, F-69140 Rilleux la Pape (FR); Greiner, Alfred, F-69450 St Cyr au Mont D'Or (FR)
(74) Mandataire: Brachotte, Charles

(56) Documents cités:
- DE-A- 2 245 518
- GB-A- 1 305 025
- GB-A- 2 180 236
- BULLETIN DE LA SOCIETE CHIMIQUE DE FRANCE, no. 8, 1969, pages 2871-2875, Paris, FR; GEORGES LE GULLANTON: "Action des agents époxydants sur les alkylidène-2 cyclopentanones"
- JOURNAL OF THE CHEMICAL SOCIETY, vol. c(20), 1971, pages 3396-3398, Letchworth, GB; D.A. WHITING: "Photodimerisation of 2-Benzyl-5-p- Bromobenzylidenecyclopentanone, a Crystal-Crystal Transformation: X-Ray Study of the Dimer"
- BERICHTE DER DEUTSCHEN CHEMISCHEN GESELLSCHAFT, vol. 120, no. 8, 8 août 1987, pages 1449-1450, Weinheim, DE; "Reaction of Cyclic Thioureas with 2- Benzylidenecycloalkanones"
- BULLETIN DE LA SOCIETE CHIMIQUE DE FRANCE, nos. 8-9, 1970, pages 2972-2980, Paris, FR; J.-M. CONIA et al.: "L'isomérisation des alcoylidène-2 cyclohexanones,-cyclopentanones et cyclobutanones par l'acide polyphosphorique"

## Description

La présente invention concerne de nouveaux composés, à usage phytosanitaire, à groupements benzylidène azolylméthylcycloalcane ou cycloalcène. Elle concerne également les procédés de préparation desdits composés et les produits éventuellement utilisables à titre d'intermédiaires dans les procédés de préparation. Elle concerne ensuite l'utilisation à titre de fongicides de ces composés, les compositions fongicides à base de ces composés et les procédés pour lutter contre les maladies fongiques des cultures utilisant ces composés. Elle concerne également un produit de multiplication des plantes cultivées qui a subi un traitement de protection par un composé de l'invention.

De nombreux produits à groupes triazole, notamment des fongicides, sont déjà connus. En particulier par les demandes de brevet EP 151084, 246982, 121979, 89100 on connait des fongicides triazoles à cycle tétrahydrofuranne. Par les demandes de brevet EP 272895, 267778, 294222 DE 3630840, BE 867245 on connait des fongicides triazoles à cycle cyclopentane. Par la demande de brevet EP 324646 et le brevet US 4684396, on connait des fongicides triazole à groupe cycloalcane. Par le brevet US 4160838 on connait des fongicides triazoles à cycle dioxolanne.

Ainsi compte tenu de cet état de la technique qui, à la connaissance du déposant, permet d'illustrer l'état de la technique selon la règle 27-1, paragraphe C, un but de la présente invention est de proposer d'autres composés fongicides à large spectre utiles notamment dans le traitement des maladies du pied comme le pietin verse ou de la feuille comme l'oîdium, la septoriose, la pyriculariose, les fusarioses, la rhynchosporiose, des maladies provoquées par les champignons pathogènes tels que Botrytis, Phoma, Aschochyta, dans des cultures aussi diverses que les céréales, la vigne, le riz, le maîs, le soja par exemple.

Ces composés sont caractérisés en ce qu'ils répondent aux formules IA ou IB ci-dessous : 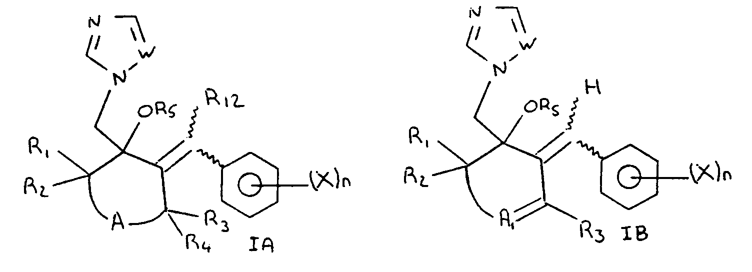
dans lesquelles
A est -CR₆ R₇- ou -CR₆ R₇ CR₈ R₉- ou - CR₆ R₇ CR₈ R₉ CR₁₀ R₁₁-
A1 est CR₇=, - CR₆ R₇- CR₉=, -CR₆ R₇-CR₈ R₉-CR₁₁=.

C'est à dire que le cycloalcane peut être un cyclopentane ou cyclohexane ou cycoheptane, ou cyclopentène, cyclohexène ou cycloheptène.
X est un atome d'halogène, de préférence le fluor, le brome, le chlore, ou un groupe cyano ou nitro, ou un groupe C₁-C₄ alkyle ou C₁-C₄ alkoxy éventuellement halogéné,
n est un nombre entier positif ou nul, inférieur à 6, les groupements X pouvant être identiques ou différents lorsque n est plus grand que 1,
W représente un groupe trivalent constitué soit d'un groupe = CH-, soit d'un atome d'azote = N-,
R₁, R₂, identiques ou différents, représentent l'atome d'hydrogène ou un radical C₁-C₄ alkyle (éventuellement substitué par un ou plusieurs atomes ou radicaux choisis parmi les atomes d'halogènes, les radicaux C₁-C₄ alkoxy, mono ou poly halo C₁-C₄ alkoxy, C₂-C₄ alcényle, C₂-C₄ alcynyle, mono ou poly halo C₂-C₄ alcényle, mono ou poly halo C₂-C₄ alcynyle) C₁-C₄ alkoxy (éventuellement substitué par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle) les radicaux C₃-C₇ cycloalkyle , C₆-C₁₀ aryle, C₇-C₁₁ aralkyle, ces divers radicaux pouvant être éventuellement substitués par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy, R₁, R₂ ensemble peuvent former une chaine C₂-C₅ hydrocarbonée constituant un cycle avec le carbone auquel R₁, R₂ sont reliés, cette chaine étant éventuellement substituée comme pour les radicaux C₆-C₁₀ aryle précédemment cités ou R₁, R₂ ensemble peuvent former une chaine C₂-C₅ hydrocarbonée dioxolane avec le carbone auquel R₁, R₂ sont reliés, cette chaine étant éventuellement substituée comme pour les radicaux C₆-C₁₀ aryle précédemment cités.
R₃, R₆ à R₁₁, identiques ou différents, représentent l'atome d'hydrogène ou un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy), les radicaux C₃-C₇ cycloalkyle, C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), ou bien deux radicaux adjacents de la chaîne A forment ensemble, avec les atomes de A auxquels ils sont rattachés, un cycle phényle accolé au cycloalcane,
R₅ représente l'atome d'hydrogène, un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle,) les radicaux C₃-C₇ cycloalkyle , C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), ou R₅ représente un groupe C(=O)-R₁₃, R₁₃ représentant un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle,) les radicaux C₃-C₇ cycloalkyle , C₆-C₁₀ aryle, C₇-C₁₁ aralkyle, ces divers radicaux pouvant être éventuellement substitués par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), un radical C₂-C₄ éthynyle, C₂-C₄ acétynyle, mono ou polyhalo C₂-C₄ éthynyle, mono ou polyhalo C₂-C₄ acétynyle,
R₁₂ a l'une des significations de R₅, à l'exception de C(=O)-R₁₃,
R₄ représente l'atome d'hydrogène, un atome d'halogène, notamment un atome de chlore ou de brome, un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle,) les radicaux C₃-C₇ cycloalkyle , C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy),

L'invention concerne également les formes salifiées des composés selon l'invention. Les formes salifiées sont les formes acceptables en agriculture parmi lesquelles on peut citer : les chlorhydrate, sulfate, oxalate, nitrate ou arylsulfonate ainsi que les complexes d'addition de ces composés avec des sels métalliques, et notamment des sels de fer, chrome, cuivre, manganèse, zinc, cobalt, étain, magnésium et aluminium.

A titre d'exemple, des complexes avec le zinc peuvent être obtenus en faisant réagir le composé de formule I avec le chlorure de zinc.

Au sens du présent texte, on entend que lorsque aucune précision n'est donnée les radicaux concernés peuvent être ramifiés ou linéaires. Le terme éventuellement halogéné signifie éventuellement mono ou poly halogéné.

Dans la formule IA ou IB le symbole 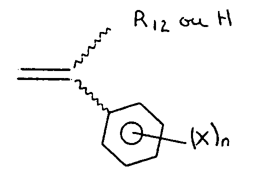
signifie que la stéréochimie de la double liaison peut être indifféremment E ou Z ou un mélange des deux. Compte tenu des contraintes stériques la forme majoritaire sera la forme où R₁₂ est en position E par rapport à R₃, R₄. La partie E est pratiquement l'unique forme obtenue lorsque R₁₂ est H.

Les composés de formule I et les composés éventuellement utilisables à titre d'intermédiaires dans les procédés de préparation et qui seront définis à l'occasion de la description de ces procédés peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

En vue des applications fongicides il a été trouvé que l'invention concernait de préférence les composés de formule IA ou IB dans laquelle n = 1, 2 ou 3, et de préférence X est un halogène choisi parmi le chlore, le brome ou le fluor.

Il a été également trouvé qu'il était préférable d'utiliser les composés de formule IA ou IB dans laquelle n = 1 ou 2, et X est un atome d'halogène placé en para lorsque n = 1 et méta para ou ortho para lorsque n = 2, de préférence n = 1 et X est placé en para.

Très avantageusement X est l'atome de chlore.

Compte tenu des restrictions définies ci-dessus prises séparemment ou en combinaisons il a été trouvé qu'il était préférable en raison des propriétés fongicides d'utiliser les composés de formule I dans laquelle W est -N=.

Compte tenu ou non des restrictions définies ci-dessus prises séparemment ou en combinaisons il a été trouvé qu'il était préférable en raison des propriétés fongicides d'utiliser les composés de formule IA ou IB dans laquelle R₃, R₆, R₈, R₁₀ sont l'atome d'hydrogène et de préférence R₄, R₇, R₉, R₁₁ sont l'atome d'hydrogène ou le radical C₁-C₄ alkyle.

Compte tenu ou non des restrictions définies ci-dessus prises séparemment ou en combinaisons il a été trouvé qu'il était préférable en raison des propriétés fongicides d'utiliser les composés de formule IA ou IB dans laquelle R₁ et R₂ sont choisis parmi les radicaux méthyle, éthyle, ou un atome d'hydrogène.

Compte tenu ou non des restrictions définies ci-dessus prises séparemment ou en combinaisons il a été trouvé qu'il était préférable en raison des propriétés fongicides d'utiliser les composés où R₅ est l'atome d'hydrogène ou C1-C4 alkyle, très avantageusement R₅ est l'atome d'hydrogène.

Compte tenu ou non des restrictions définies ci-dessus prises séparemment ou en combinaisons il a été trouvé qu'il était préférable en raison des propriétés fongicides d'utiliser les composés où R₁₂ est l'atome d'hydrogène.

Compte tenu ou non des restrictions définies ci-dessus prises séparemment ou en combinaisons il a été trouvé qu'il était préférable en raison des propriétés fongicides d'utiliser les composés de formule IA, de préférence où A est CR₆R₇ ou -CR₆R₇CR₈R₉.

De préférence pour leur activité foliaire (notamment anti-botrytis) et/ou leur absence de phytotoxicité ce qui leur permet d'être appliqués en traîtement de semences, on utilisera les triazoles de formule IA où A est CR₆R₇, R₁, R₂ sont choisis parmi les radicaux méthyle ou éthyle, R₃, R₅ à R₇, R₁₂ sont l'atome d'hydrogène, R₄ est méthyle, éthyle, n-propyle, i-propyle, ou l'atome d'hydrogène, ou A est CR₆R₇ CR₈R₉, R₁, R₂ sont choisis parmi les radicaux méthyle, éthyle, ou l'atome d'hydrogène, R₃, R₅ à R₉, R₁₂ sont l'atome d'hydrogène, R₄ est méthyle, éthyle, n-propyle, i-propyle, ou l'atome d'hydrogène.

On préfère les composés suivants :
- 2-(4-chlorobenzylidène)-5-méthyl 5-éthyl 1-(1H-1,2,4-triazol-1-ylméthyl)-1-cyclopentanol
- 2-(4-chlorobenzylidène)-5,5-diméthyl 1-(1H-1,2,4-triazol-1-ylméthyl)-1-cyclopentanol
- 2-(4-chlorobenzylidène)-6-méthyl 1-(1H-1,2,4-triazol-1-ylméthyl)-1-cyclohexanol
- 2-(4-chlorobenzylidène)-6,6-diméthyl 1-(1H-1,2,4-triazol-1-yl-méthyl)-1-cyclohexanol.
- 2-(4-chlorobenzylidène)-1-(1H-1,2,4-triazol-1-ylméthyl)-1-cyclohexanol.

La présente invention concerne également des procédés de préparation des composés selon l'invention.

Dans l'exposé qui va suivre les substituants décrits ci-dessous ont la même signification que ceux indiqués plus haut, sauf indication contraire.

### A- Cas des composés de formule IA

Ce procédé consiste à faire réagir le chlorure d'un acide oméga-alcénoique de formule : 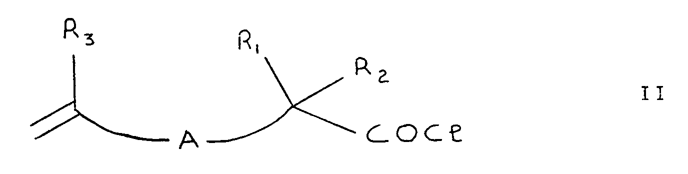
dans laquelle R₁, R₂ ne peuvent être un radical C₁-C₄ alkyle substitué par un ou plusieurs radicaux C₂-C₄ alcényle, C₂-C₄ alcynyle, mono ou poly halo C₂-C₄ alcényle, mono ou poly halo C₂-C₄ alcynyle ou un radical C₁-C₄ alcoxy, en présence de chlorure d'aluminium dans un solvant inerte comme le dichlorométhane ou le disulfure de carbone ou le nitrométhane comme décrit dans la littérature par K.R KOPECKY et al dans Can.J.Chem. 59, 3273 (1981) et W.C. AGOSTA et al J.Am.Soc 93, 5513 (1971) de maniére à obtenir un mélange de cycloalcanone et cycloalcènone de formule: 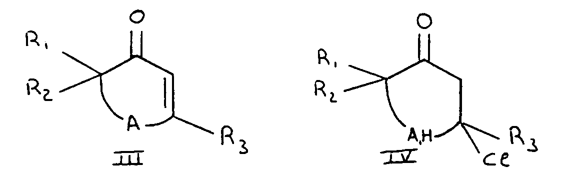
Dans le composé de formule IV, A1H correspond aux groupes -CR₇H, ou -CR₆R₇-CR₉H ou -CR₆R₇-CR₈R₉-CR₁₁H, et n'est obtenu que lorsque A est donc A1H.

Dans le cas où l'on veut aboutir à un composé de formule V où R₄ est l'atome d'hydrogène, le composé de formule III est ensuite soumis à une hydrogénation catalytique pour obtenir le composé : 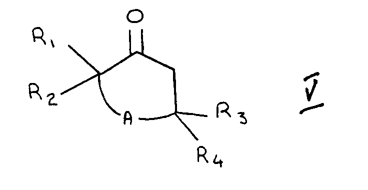
Dans le cas où l'on veut aboutir à un composé de formule V ci-dessus où R₄ est différent de l'atome d'halogène ou d'hydrogène, l'on fait réagir un magnésien de formule R₄M (M = MgHal), obtenu de manière connue en faisant réagir par exemple le composé de formule R₈I avec du magnésium en présence d'éther, avec une cycloalcénone de formule III en présence d'une quantité catalytique d'iodure de cuivre à basse température, dans un solvant polaire de manière connue. On ajoute ensuite an mélange de l'eau afin d'isoler le composé de formule V.

La cycloalcanone ainsi obtenue de formule (V), dans laquelle rappelons le, R₁, R₂ ont la même signification que dans la formule générale à l'exception qu'ils ne peuvent être un radical C₁-C₄ alkyle substitué par un ou plusieurs radicaux C₂-C₄ alcényle, C₂-C₄ alcynyle, mono ou poly halo C₂-C₄ alcényle, mono ou poly halo C₂-C₄ alcynyle, ou le radical C₁-C₄ alcoxy, R₄ a la même signification que dans la formule générale à l'exception qu'il ne peut être l'atome d'halogène, R₃, R₆, R₇ ont la même signification que dans la formule générale, est soumise à la réaction bien connue d'aldolisation crotonisation par condensation avec un benzaldehyde de formule : 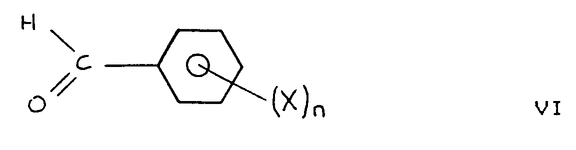
afin d'obtenir des composés de formule VII où R₁₂ est l'atome d'hydrogène : 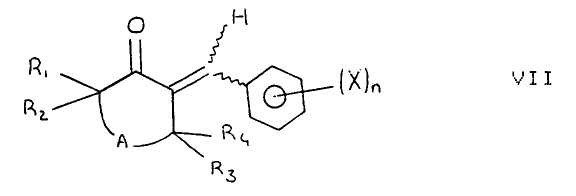
Pour obtenir un composé de formule VII où R₁₂ est différent de l'atome d'hydrogène l'on fait réagir un éther silylé de formule XI : 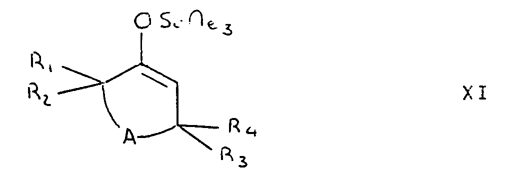
avec un cétal de formule XII : 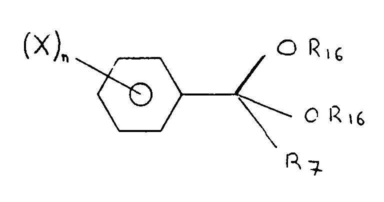
dans laquelle R₁₆ est un radical C₁-C₄ alkyle par mélange préalable du cétal de formule XII avec du tétra chlorure de titane dans un solvant halogéné comme le dichlorométhane à 0°C environ puis ajout de l'éther silylé de formule XI à 0°C environ et hydrolyse subséquente avec HCl comme décrit dans T. NUKAIYANA et al J. Am. Chem. Soc. 1974, 96, 7503.

Pour obtenir un composé de formule VII dans laquelle R₁ est un groupe alkyle ou aralkyle, éventuellement substitué, tel que défini dans la formule générale, R₂ différent d'hydrogène ou du radical C₁-C₄ alkyle susbtitué par un ou plusieurs radicaux C₂-C₄ alcényle, C₂-C₄ alcynyle, mono ou poly halo C₂-C₄ alcynyle, un autre procédé consiste à faire réagir une des cétones de formule III, V, VII préparées comme ci-dessus, où R₁ est l'atome d'hydrogène et R₂ est différent de l'atome d'hydrogène ou du radical C₁-C₄ alkyle substitué par un ou plusieurs radicaux C₂-C₄ alcényle, C₂-C₄ alcynyle, mono ou poly halo C₂-C₄ alcynyle, avec un agent alkylant R₁-Y où R₁ est un groupe alkyle ou aralkyle, éventuellement substitué, tel que défini dans la formule générale, et Y un groupe partant tel que halogène, sulfonate ou sulfate par exemple en présence d'une base organique ou minérale, de préférence les hydroxydes, alcoolates et hydrures alcalins ou alcalinoterreux dans un solvant ou mélange de solvants protiques ou aprotiques tels les hydrocarbures saturés, insaturés ou aromatiques, éventuellement halogénés, les alcools, les amides, les nitriles, les solvants dérivés oxygénés de sulfures tels DMSO ou le sulfolane. On obtient ainsi d'autres cétones de formule III, V, VII. Dans le cas où l'on a obtenu des cétones de formule III ou V ce qui est possible dans le cas d'une des voies présentées ci-dessus on suit ensuite la procédure de transformation indiquée ci-dessus pour atteindre les cétones de formule VII.

Pour obtenir un composé de formule VII dans laquelle R₁, R₂ identiques sont un groupe alkyle ou aralkyle tel que définis dans la formule générale, un autre procédé consiste à faire réagir une cétone de formule III, V, VII préparée comme ci-dessus, où R₁, R₂ sont l'atome d'hydrogène avec un agent alkylant R₁-Y où R₁ est un groupe alkyle ou aralkyl tel que définis dans la formule générale, et Y un groupe partant tel que halogène, sulfonate ou sulfate par exemple en présence d'une base organique ou minérale, de préférence les hydroxydes, alcoolates et hydrures alcalins ou alcalinoterreux dans un solvant ou mélange de solvants protiques ou aprotiques tels les hydrocarbures saturés, insaturés ou aromatiques, éventuellement halogénés, les alcools, les amides, les nitriles, les solvants dérivés oxygénés de sulfures tels le DMSO ou le sulfolane. Il est parfois possible d'isoler l'intermédiaire où l'un seulement de R₁, R₂ est un groupe alkyle ou aralkyle tel que défini plus haut. Ces composés VII sont alors aussi utilisables pour obtenir selon les procédés décrits les composés I. Dans le cas où l'on a obtenu des cétones de formule III ou V ce qui est possible dans le cas d'une des voies présentées ci-dessus on suit ensuite la procédure de transformation indiquée ci-dessus pour atteindre les cétones de formule VII.

Dans le cas où R₁, R₂ forment une chaine C₂-C₅ hydrocarbonée, on fait réagir de la même façon que précédemment un composé de formule Y-R₉-Y, R₉ étant un radical C₂-C₅ hydrocarboné éventuellement substitué (par exemple par un ou plusieurs atomes ou radicaux tels que les atomes d'halogènes, les radicaux C₁-C₄ alkyle, les radicaux mono ou polyhalo C₁-C₄ alkyle, les radicaux C₁-C₄ alkoxy et les radicaux mono ou poly halo C₁-C₄ alkoxy) sur une cétone de formule III, V, VII, où R₁, R₂ sont l'atome d'hydrogène, selon le procédé indiqué ci-dessus.

Dans le cas où R₁ et/ou R₂ sont un groupement allyle un autre procédé consiste à faire réagir une cétone de formule V dans laquelle R₁, R₂ sont l'atome d'hydrogène avec 2 moles d'alcool allylique, 1 mole de 2,2-diméthoxypropane, en présence d'une quantité catalytique d'acide paratoluènesulfonique et en solvant inerte comme le toluène afin d'obtenir la cétone monoallylée correspondante comme cela est bien décrit par W.L. Howard N.B. Lorette Org. Synth. 42, 34, (1964). Cette cétone est mise ensuite à réagir avec le composé de formule VI comme indiqué précédemment et addition d'un autre radical allyle par la méthode indiquée précédemment par alkylation.

Dans le cas où R₁ et/ou R₂ sont un radical C₁-C₄ alkoxy il est avantageux de partir d'une cycloalcanone de formule V où le/les radicaux alcoxy sont déjà introduits au préalable par une méthode connue de l'homme de l'art (par exemple la réaction des cétones α bromées avec les alcolates alcalins).

Un autre procédé général d'obtention des cétones VII pour lesquelles l'un au moins de R₁, R₂ est l'hydrogène consiste a préparer une énamine à partir d'une cycloalcanone V où l'un au moins de R₁ et R₂ est l'hydrogène selon B.C. Mc KUSICK, F.E. NURFORD Org. Synth. Coll. Vol. V, 808 et de condenser cette dernière avec un benzaldéhyde VI selon L. BIRKOFFER, S.M. KIM, H.D. ENGELS Chem. Ber. (1962) 95, 1495. L'hydrolyse acide selon cet article conduit alors aux cétones VII où l'un au moins des groupes R₁, R₂ est l'hydrogène.

Le dit composé de formule (VII) est mis à réagir avec un ylure de sulfonium comme décrit dans E.J.COREY M. CHAYKOVSKY, J.Am.Chem. Soc 87, 1313, (1965) afin de conduire aux oxirannes de formule: 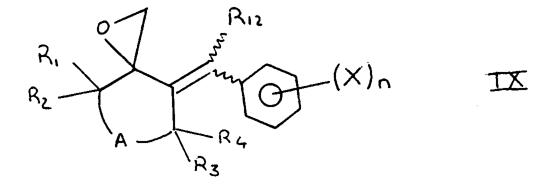
On fait réagir ensuite l'oxiranne de formule (IX) avec un imidazole ou un triazole non substitué en présence de base organique ou minérale par exemple la pyridine, la triéthylamine, la soude, la potasse, les carbonates et bicarbonates de métaux alcalins ou alcalino terreux et les hydrures de métaux alcalins et dans un solvant approprié tel que par exemple les alcools, les cétones, les amides, les nitriles, les hydrocarbures aromatiques éventuellement halogénés, à une température comprise entre 80° et le reflux du solvant et dans un rapport molaire IX : imidazole ou triazole de préférence compris entre 1,1 et 0,2, ce qui conduit aux composés de formule I dans laquelle R₅ est l'atome d'hydrogène et R₄ est différent de l'atome d'halogène, les autres susbtituants ayant la même signification que celle indiquée dans la formule générale.

Les composés de formule I où R₄ représente un atome d'halogène et R₅ est l'atome d'hydrogène sont obtenus par halogénation allylique des composés de formule I dans laquelle R₄, R₅ sont l' atome d'hydrogène avec NBS (N-bromosuccinimide), NCS (N-chlorosuccinimide), t-BuOCl dans CCl4 en présence de peroxydes ou de lumière UV selon L. HORNER, E.H WINKELMANN, Angew.Chem.71, 349, (1959).

Le composé de formule I dans laquelle R₅ est différent de l'atome d'hydrogène et R₄ est différent de l'atome d'halogène est obtenu par étherification ou estérification des composés de formule I dans laquelle R₅ est l'atome d'hydrogène et R₄ est différent de l'atome d'halogène selon de méthodes conventionnelles bien connues de l'homme de l'art : les éthers peuvent être obtenus en traitant un sel alcalin de l'alcool de formule (I) (par exemple un sel de lithium ou de sodium avec l'halogénure approprié de formule R₅Hal. Les esters peuvent être obtenus de manière conventionnelle en traitant un sel de métal alcalin de formule (I) avec le chlorure d'acide approprié de formule R₆C=OCl ou l'anhydride correspondant de formule (R₆)₂O₂C=O.

Les composés de formule (I) dans laquelle R₄ est l'atome d'halogène et R₅ est différent de l'atome d'hydrogène sont obtenus par dans une première étape estérification ou éthérification comme décrit précédemment d'un composé de formule (I) dans laquelle R₅, R₄ sont l'atome d'hydrogène puis halogénation du composé résultant de formule (I) comme décrit précédemment par NBS par exemple.

Les composés de formule XI et XII sont obtenus de manière connue de l'homme de l'art. Par exemple, on peut acétaliser la benzophénone correspondante en milieu acide avec un alcool R₁₀OH dans le cas du composé de formule XII. On peut additionner sur la cyclopentanone correspondante le chlorure de triméthylsilyl en présence de diméthylformamide et de triéthylamine dans le cas composé de formule XII.

### B- Cas des composés de formule IB

La cétone cyclique de formule (IV) est soumise à la réaction bien connue d'aldolisation crotonisation par condensation avec un benzaldehyde de formule VI afin d'obtenir le composé de formule : 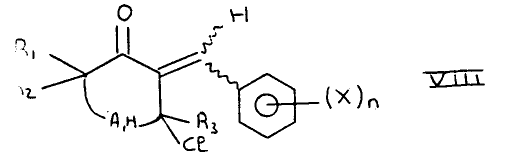
Le dit composé de formule (VIII) est mis à réagir avec un ylure de sulfonium comme décrit dans E.J.COREY Michael CHAYKOVSKY J.Am.Chem.Soc.87, 1313, (1965) afin de conduire à l'oxiranne de formule: 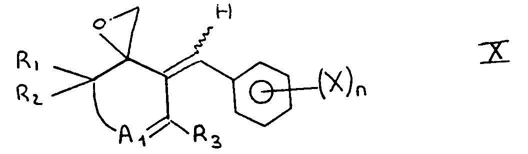
On fait réagir l'oxiranne de formule ( X) avec un imidazole ou un triazole non substitué en présence de base organique ou minérale par exemple la pyridine, la triéthyalmine, la soude, la potasse, les carbonates et bicarbonate de métaux alcalins et dans un solvant approprié tel que par exemple les alcools, les cétones, les amides, les nitriles, les hydrocarbures aromatiques éventuellement halogénés, à une température comprise entre 80°C et le reflux du solvant et dans un rapport molaire IX : imidazole ou triazole de préférence compris entre 1,1 et 0,2, ce qui conduit aux composés de formule IB : 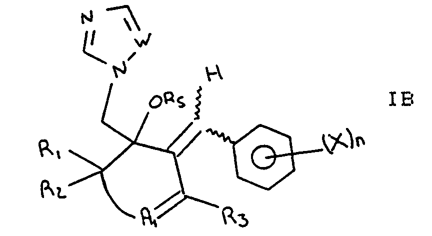
dans laquelle R₅ = H.

Les mêmes méthodes pour alkyler ou allyler la position en α de l'hydroxy (R₁, R₂) que celles indiquées précédemment s'appliquent de même que celles pour obtenir un composé où R₅ différent de H.

Bien entendu d'autres procédés de préparation peuvent également convenir.

L'invention a également pour objet les composés éventuellement utilisables à titre d'intermédiaires dans les procédés de préparation décrit ci-dessus et de formule III, IV, V, VII, VIII, IX, X décrites ci-avant, dans lesquelles:
- les substituants n, X, R₁, R₂, A₁, R₄ A, R₃, R₁₂ ont la même signification que dans les formules IA ou IB,
- et le groupe A₁H de la formule VIII correspond aux groupes -CR₇H, ou -CR₆R₇-CR₉H ou - -CR₆R₇-CR₈R₉-CR₁₁H, dans lesquels les substituants R₆, R₇, R₈, R₉, R₁₁, ont la même signification que dans les formules IA ou IB,
sauf les composés suivants :
2-benzylidènecyclopentanone,
2-(4-chlorobenzylidène) cyclopentanone,
2-(4-chlorobenzylidène)-5-benzylcyclopentanone,
2-(4-bromobenzylidène)-5-benzylcyclopentanone,
2-benzylidène-3-méthylcyclopentanone,
2-benzylidène-5-méthyl-cyclopentanone,
2-benzylidène-5-benzylcyclopentanone,
2-benzylidènecyclohexanone,
6-cyclohexyl-2-benzylidènecyclohexanone,
2-benzylidène-5-méthyl-6-cyclohexylcyclohexanone,
2-(4-méthoxybenzylidène)-4-méthyl-6-phénylcyclohexanone,
2-benzylidène-6(4-isopropylphényl)cyclohexanone,
2-benzylidène-6-méthyl-cyclohexanone,
2-benzylidène-4-méthyl-cyclohexanone,
2-benzylidènecycloheptanone,
2-benzylidène-6-benzyl-cyclohexanone ;
et à l'exclusion des composés de formule VII, dans laquelle :
- R₁ est un atome d'hydrogène ; et
- R₂ est un atome d'hydrogène ou un radical cyclohexyle ou phényle, ledit radical étant éventuellement substitué par un radical C₁-C₄ alkyle ; et
- R₃ est un atome d'hydrogène ; et
- R₄ est un atome d'hydrogène ou un radical méthyle, éthyle, ou propyle ; et
- A est -CHR₆-, -CHR₆-CHR₈-, -CHR₆-CHR₈-CHR₁₀- ; et
- R₆, R₈, R₁₀ sont choisis parmi un atome d'hydrogène, ou un radical méthyle, éthyle, ou propyle ; et
- n égale 0, 1 ou 2 ; et
- X est un atome d'hydrogène ou d'halogène, un radical C₁-C₄ alkyle ou C₁-C₄ alkoxy, ou un groupe nitro.

La présente invention concerne également l'utilisation des composés de formule I à titre de fongicides.

Les composés selon l'invention peuvent être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, fusarium roseum, fusarium nivale, helminthosporioses, rhynchosporioses, septorioses, rhizoctones des végétaux et des plantes en général et en particulier des céréales telles que le blé, l'orge, le seigle, l'avoine et leurs hybrides et aussi le riz et le maïs. Les composés selon l'invention sont actifs en particulier contre les champignons notamment de type basidiomycètes, ascomycètes, adelomycètes ou fungi-imperfecti comme Botrytis cinerea, Erysiphe graminis, Puccinia recondita, Piricularia oryzae, Cercospora beticola, Puccinia striiformis, Erysiphe cichoracearum, Fusarium oxysporum (melonis), Pyrenophora avenae, Septoria tritici, Venturia inaequalis, Whetzelinia sclerotiorum, Monilia laxa, Mycosphaerella fijiensis, Marssonina panettoniana, Alternaria solani, Aspergillus niger, Cercospora arachidicola, Cladosporium herbarum, Helminthosporium oryzae, Penicillium expansum, Pestalozzia sp, Phialophora cinerescens, Phoma betae, Phoma foveata, Phoma lingam, Ustilago maydis, Verticillium dahliae, Ascochyta pisi, Guignardia bidwellii, Corticium rolfsii, Phomopsis viticola, Sclerotinia sclerotiorum, Sclerotinia minor, Coryneum cardinale, Rhizoctonia solani.

Ils sont aussi et encore actifs contre les champignons suivants : Acrostalagmus koningi, les Alternaria, les Colletotrichum, Corticium rolfsii, Diplodia natalensis, Gaeumannomyces graminis, Gibberella fujikuroi, Hormodendron cladosporioides, Lentinus degener ou tigrinus, Lenzites quercina, Memnoniella echinata, Myrothecium verrucaria, Paecylomyces varioti, Pellicularia sasakii, Phellinus megaloporus, Polystictus sanguineus, Poria vaporaria, Sclerotium rolfsii, Stachybotris atra, les Stereum, Stilbum sp. Trametes trabea, Trichoderma pseudokoningi, Trichothecium roseum.

Les composés de l'invention sont spécialement intéressants par leur spectre large au niveau des maladies des céréales (oïdium, rouille, piétin-verse, helminthosporioses, septorioses et fusarioses). Ils présentent également un grand intérêt en raison de leur activité sur la pourriture grise (Botrytis) et les cercosporioses, et, de ce fait, ils peuvent être appliqués sur des cultures aussi variées que la vigne, les cultures maraîchères et l'arboriculture et les cultures tropicales telles que l'arachide, le bananier, le caféier, la noix de pécan et d'autres.

Vu leur absence de phytotoxicité les composés peuvent être utilisés pour la protection des produits de multiplication des plantes contre les maladies causées par des champignons.

L'invention concerne donc en outre un produit de multiplication des plantes cultivées qui a subi un traitement de protection par un composé de l'invention.

On désigne par le nom "produit de multiplication" toutes les parties génératives de la plante qu'on peut utiliser pour la multiplication de celle-ci. Ce "produit de multiplication" est choisi parmi : les graines (semences au sens étroit), les racines, les fruits, les tubercules, les bulbes, les rhizomes, les parties de tige, les plants, (pousses) et autres parties de plantes, les plantes germées et les jeunes plants qui doivent être transplantés aprés germination ou aprés la sortie de terre. Ces jeunes plants peuvent être protégés avant la transplantation par un traitement total ou partiel par immersion.

En général, ces composés seront appliqués à raison de 0,1 g à 500 g par quintal de graines.

Ainsi, ces composés peuvent être utilisés dans le traitement des semences (par exemple céréales, cotonnier, betterave, colza, graines fourragères, grains légumières) par exemple sous la forme d'enrobage ou de pelliculage. On pourra trouver dans US 3,989,501, col 7, l 17-23, une forme d'application. De même, dans FR-A-2 588 442. On pourra également utiliser des suspensions concentrées.

En général ces formulations sont déja connues voir par exemple "catalogue of pesticide formulation types and international coding system" édité par le GIFAP technical monograph n°2, pages 12 à 14, révisée en janvier 1984.

Outre les applications déjà décrites plus haut, les produits selon l'invention présentent en outre une excellente activité biocide à l'égard de nombreuses autres variétés de microorganismes parmi lesquelles on peut citer à titre non limitatif, des champignons comme ceux des genres :
- Pullularia comme l'espèce P. pullulans,
- Chaetomium comme l'espèce C. globosum,
- Aspergillus comme l'espèce Aspergillus niger,
- Coniophora comme l'espèce C. puteana.

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels.

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Les composés selon l'invention utilisés seuls ou sous la forme de compositions telles que définies ci-dessus dans les traitements du bois, sont généralement mis en oeuvre avec des solvants organiques et peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole, les insecticides tels que les pyrethroïdes ou les organochlorés.

L'invention concerne également un procédé de traitement des cultures atteintes ou susceptibles d'être atteintes par les maladies fongiques caractérisé en ce que l'on applique sur les feuilles une dose efficace d'un composé de l'invention.

Les composés s'appliquent avantageusement à des doses de 0,002 à 5 kg/ha, et plus spécifiquement de 0,005 à 1 kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et/ou les agents tensio-actifs également acceptables en agriculture. En particulier sont utilisables les supports inertes et usuels et les agents tensio-actifs usuels.

Ces compositions contiennent habituellement entre 0,5 et 95% de composé selon l'invention.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Ces compositions peuvant contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associésà tous les additifs solides ou liquides correspondant aux techniques habituelles de la mise en formulation.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Selon un exemple de composition de granulés, on utilise les constituants suivants :

### Exemple F 9

| | |
|---|---|
| - matière active | 50 g |
| - épichlorhydrine | 2,5 g |
| - éther de cétyle et de polyglycol | 2,5 g |
| - polyéthylène glycol | 35 g |
| - kaolin (granulométrie : 0,3 à 0,8 mm) | 910 g |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudre pour poudrage ; on peut aussi utliliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active.

Par exemple, en plus du solvant, les concentrés émulsionnables peuvant contenir quand c'est nécessaire, 2 à 20 % d'addifits appropriés comme les stabilisants, les agents tensio-actifs, les agents de pénétration, les inhibiteurs de corrosion, les colorants ou les adhésifs précedemment cités.

A titre d'exemple, voici la composition de quelques concentrés :

### Exemple F (formulation) 1

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique q.s.p | 1 litre |

Selon une autre formule de concentré émulsionnable, on utilise :

### Exemple F 2 :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

### Exemple F 3 :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Une autre composition de poudre à pulvériser à 70 % utilise les constituants suivants :

### Exemple F 4 :

| | |
|---|---|
| - matière active | 700 g |
| - dibutylnaphtylsulfonate de sodium | 50 g |
| - produit de condensation en proportions 3/2/1 d'acide naphtalène sulfonique, d'acide phénolsulfonique et de formaldéhyde | 30 g |
| - kaolin | 100 g |
| - craie de champagne | 120 g |

Une autre composition de poudre à pulvériser à 40 % utilise les constituants suivants :

### Exemple F 5 :

| | |
|---|---|
| - matière active | 400 g |
| - lignosulfonate de sodium | 50 g |
| - dibutylnaphtalène sulfonate de sodium | 10 g |
| - silice | 540 g |

Une autre composition de poudre à pulvériser à 25 % utilise les constituants suivants :

### Exemple F 6 :

| | |
|---|---|
| - matière active | 250 g |
| - lignosulfonate de calcium | 45 g |
| - mélange équipondéral de craie de Champagne et d'hydroxyéthylcellulose | 19 g |
| - dibutylnaphtalène sulfonate de sodium | 15 g |
| - silice | 195 g |
| - craie de Champagne | 195 g |
| - kaolin | 281 g |

Une autre composition de poudre à pulvériser à 25 % utilise les constituants suivants :

### Exemple F 7 :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |
| - aluminosilicate de sodium | 543 g |
| - kieselguhr | 165 g |

Une autre composition de poudre à pulvériser à 10 % utilise les constituants suivants :

### Exemple F 8 :

| | |
|---|---|
| - matière active | 100 g |
| - mélange de sels de sodium de sulfates d'acides gras saturés | 30 g |
| - produit de condensation d'acide naphtalène sulfonique et de formaldéhyde | 50 g |
| - kaolin | 820 g |

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les doses d'emploi dans le case d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de 5x10-5 à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de 5.10-5% à 95 % (en poids).

Les exemples suivants illustrent des modes particuliers de préparation de composés selon l'invention ainsi que ces composés eux-mêmes.

La nomenclature des composés à été indiquée selon les normes françaises mis à part le fait que la numérotation des substituants a été mise avant les substituants eux-mêmes.

### Exemple I : Préparation de 2-(4-chlorobenzylidène)5,5-diméthyl 1-(1H-1,2,4-triazol-1-ylméthyl)-1-cyclopentanol

A un mélange de 10g de 2,2-diméthyl cyclopentanone et 13,8g de 4-chlorobenzaldehyde dans 100ml d'éthanol à 0°C, est ajouté 100ml d'une solution aqueuse de soude à 10%. Aprés 30 minutes une épaisse bouillie était filtrée et le solide lavé puis séché. On obtient 12,5g de 2,2-diméthyl 5-(4-chloro benzylidène) 1-cyclopentanone de point de fusion 120°C. Ce composé dissout dans 50ml de THF était ajouté à une solution formée de la facon suivante: 1,9 g d'hydrure de sodium (dispersion à 80% dans l'huile minérale) dans 50ml de DMSO anhydre est chauffée à 80°C jusqu'à dissolution totale du solide. Puis la solution est diluée avec 100ml de THF puis refroidi à -10°C. On ajoute au mélange en dix minutes une solution de 11,5g de triméthylsulfonium iodure dans 80ml de diméthylsulfoxide et le mélange était agité pendant 15 minutes à -10°C. Une solution de 11,8g de 2,2-diméthyl 4-chloro 5-(4-chloro benzylidène) 1-cyclopentanone était ensuite ajouté dans 100ml de THF.

Le mélange ainsi produit est laissé à température ambiante puis versé dans l'eau et extrait à l'éther, lavé à l'eau, séché, distillé. On obtient le 7-(4-chlorobenzylidène) 4,4-diméthyl 1-oxaspiro (2,4) heptane directement utilisé pour l'étape suivante.

Un mélange de 5g du produit avec 2,8g de 1,2,4-triazole et 11g de carbonate de potassium est chauffé dans 40ml de N,N diméthylformamide durant 4 heures. Le mélange est versé dans l'eau, extrait à l'acétate d'éthyle. La phase organique est lavée, séchée, recristallisée pour obtenir le produit annoncé dont le point de fusion est de 154°C (composé n° 1).

De la même façon ont été obtenus les composés suivants:
2-(4-fluorobenzylidène) 5,5-diméthyl 1-(1H-1,2,4-triazol -1-ylméthyl)-1-cyclopentanol (P.F 147°C) composé n° 2
2-(2,4-dichlorobenzylidène) 5,5-diméthyl 1-(1H-1,2,4-triazol -1-ylméthyl)-1-cyclopentanol (P.F 130°C) composé n° 3
2-(4-trifluorométhylbenzylidène) 5,5-diméthyl 1-(1H-1,2,4-triazol-1-ylméthyl)-1-cyclopentanol (P.F 132°C) composé n° 4
2-(4-chlorobenzylidène) 1-(1H-1,2,4-triazol-1-ylméthyl)-1-cyclopentanol (huile) composé n° 5
2-benzylidène 1-(1H-1,2,4-triazol-1-ylméthyl)-1-cyclopentanol (huile) composé n° 6
2-(4-bromobenzylidène) 5,5-diméthyl 1-(1H-1,2,4-triazol-1-ylméthyl)-1-cyclopentanol (P.F 152°C) composé n° 7
2-benzylidène 5,5-diméthyl 1-(1H-1,2,4-triazol-1-ylméthyl)-1-cyclopentanol (P.F 137°C) composé n° 8
2-(3,4-dichlorobenzylidène) 5,5-diméthyl 1-(1H-1,2,4-triazol-1-ylméthyl)-1-cyclopentanol (P.F 157°C) composé n° 9
2-(4-chlorobenzylidène) 5-méthyl 1-(1H-1,2,4-triazol-1-ylméthyl)-1-cyclopentanol (huile) composé n° 10
2-(2-chlorobenzylidène) 5,5-diméthyl 1-(1H-1,2,4-triazol-1-ylméthyl)-1-cyclopentanol (huile) composé n° 11
2-(4-phénylbenzylidène) 5,5-diméthyl 1-(1H-1,2,4-triazol-1-ylméthyl)-1-cyclopentanol (P.F 167°C) composé n° 12
2-(4-chlorobenzylidène) 5-méthyl 5-éthyl 1-(1H-1,2,4-triazol-1-ylméthyl)-1-cyclopentanol (P.F 144 °C) composé n° 13
2-(4-chlorobenzylidène) 5,5-diméthyl 1-(1H-1,2,4-triazol-1-ylméthyl)-1-méthoxy-cyclopentane (P.F 84°C) composé n° 14
2-(4-chlorobenzylidène) 4,5,5-triméthyl 1-(1H-1,2,4-triazol-1-ylméthyl)-1-cyclopentanol (P.F 148°C°) composé n° 15
2-(4-chlorobenzylidène) 5-méthyl 5-méthoxyméthyl 1-(1H-1,2,4-triazol-1-ylméthyl)-1-cyclopentanol (P.F 109°C) composé n° 16
2-(4-chlorobenzylidène) 5,5-di n-propyl 1-(1H-1,2,4-triazol-1-ylméthyl)-1-cyclopentanol (P.F 123°C) composé n° 17
2-(4-chlorobenzylidène) 5,5-diéthyl 1-(1H-1,2,4-triazol-1-ylméthyl)-1-cyclopentanol (P.F 171°C) composé n° 18
2-(4-fluorobenzylidène) 5,5-diéthyl 1-(1H-1,2,4-triazol-1-ylméthyl)-1-cyclopentanol (P.F 166°C) composé n° 19
2-(4-fluorobenzylidène) 5,5-di n-propyl 1-(1H-1,2,4-triazol-1-ylméthyl)-1-cyclopentanol (P.F 127°C) composé n° 20
2-(4-cyanobenzylidène) 5,5-diméthyl 1-(1H-1,2,4-triazol-1-ylméthyl)-1-cyclopentanol (P.F 137°C) composé n° 21
2-(4-chlorobenzylidène) 5,5-diméthyl 3-éthyl 1-(1H-1,2,4-triazol-1-ylméthyl)-1-cyclopentanol (P.F 58°C) composé n° 22
2-(4-parachlorophénoxybenzylidène) 5,5-diméthyl 1-(1H-1,2,4-triazol-1-ylméthyl)-1-cyclopentanol (P.F 112°C) composé n° 23
2-(4-chlorobenzylidène) 3-isopropyl 5,5-diméthyl 1-(1H-1,2,4-triazol-1-ylméthyl)-1-cyclopentanol (huile) composé n° 24

### Exemple II: Préparation de 2-(4-chlorobenzylidène) 5,5-diméthyl 1-(1H-imidazolylméthyl)-1-cyclopentanol

Un mélange de 5g de 7-(4-chlorobenzylidène) 4,4-diméthyl 1-oxaspiro (2,4)heptane produit selon l'exemple précédent avec 2,8g de l'imidazole et 11g de carbonate de potassium est chauffé dans 40ml de N,N diméthylformamide durant 4 heures. Le mélange est versé dans l'eau, extrait à l'acétate d'éthyle. La phase organique est lavée, séchée, recristallisée pour obtenir le produit annoncé dont le point de fusion est de 174°C, composé n°25.

De la même facon ont été obtenus les composés suivants:
2-(4-fluorobenzylidène) 5,5-diméthyl 1-(1H-imidazolyl méthyl)-1-cyclopentanol (P.F 160°C) composé n° 26
2-(2,4-dichlorobenzylidène) 5,5-diméthyl 1-(1H-imidazolyl méthyl)-1-cyclopentanol (P.F 134°C) composé n° 27
2-(4-trifluorométhylbenzylidène) 5,5-diméthyl 1-(1H-imidazolylméthyl)-1-cyclopentanol (P.F 171°C) composé n° 28

### Exemple III: Préparation de 2-(4-fluorobenzylidène) 5,5-diméthyl 1-(1H-1,2,4-triazol-1-ylméthyl)-1-hydroxy 3-cyclopentène

A un mélange de 10g de 2,2-diméthyl 4-chloro 1-cyclo pentanone et 8,4g de 4-fluorobenzaldehyde dans 100ml d'éthanol à 0°C, est ajouté 50ml d'une solution aqueuse de soude à 10%. Aprés 30 minutes une épaisse bouillie était filtrée et le solide lavé puis séché. On obtient 11,8g de 2,2-diméthyl 4-chloro 5-(4-fluoro benzylidène) 1-cyclopentanone de point de fusion 69°C. 3,1g d'hydrure de sodium (dispersion à 80% dans l'huile minérale) dans 50ml de DMSO anhydre est chauffée à 80°C jusqu'à dissolution totale du solide. Puis la solution est diluée avec 100ml de THF puis refroidi à -10°C. On ajoute au mélange en dix minutes une solution de 11,5g de triméthylsulfonium iodure dans 80ml de diméthylsulfoxide et le mélange était agité pendant 15 minutes à -10°C. Une solution de 11,8g de 2,2-diméthyl 4-chloro 5-(4-fluoro benzylidène) 1-cyclopentanone était ensuite ajouté dans 100ml de THF. Le mélange ainsi produit est laissé à température ambiante puis versé dans l'eau et extrait à l'éther, lavé à l'eau, séché, distillé. On obtient le 7-(4-fluorobenzylidène) 4,4-diméthyl 1-oxaspiro (2,4) 5-heptène directement utilisé pour l'étape suivante.

Un mélange de 5,5g du produit obtenu avec 2g de 1,2,4-triazole et 6,6g de carbonate de potassium est chauffé dans 50ml de N,N diméthylformamide durant 4 heures. Le mélange est versé dans l'eau, extrait à l'acétate d'éthyle. La phase organique est lavée, séchée, recristallisée pour obtenir 2,4g du produit annoncé dont le point de fusion est de 168°C, composé n°29.

De la même facon ont été obtenus les composés suivants:
2-(4-chlorobenzylidène) 5,5-diméthyl 1-(1H-1,2,4-triazol-1-ylméthyl)-1-hydroxy 3-cyclopentène (point de fusion 203°C) composé n° 30
2-(2,4-dichlorobenzylidène) 5,5-diméthyl 1-(1H-1,2,4-triazol-1-ylméthyl)-1-hydroxy 3-cyclopentène (point de fusion 182°C) composé n° 31

### Exemple IV : Préparation de 2-(4-fluorobenzylidène) 5,5-diméthyl 1-(1H-imidazolylméthyl)-1-hydroxy 3-cyclopentène

Un mélange de 5,5g de 7-(4-fluorobenzylidène) 4,4-diméthyl 1-oxaspiro (2,4) 5-heptène produit selon l'exemple précédent avec 2g de l'imidazole et 6,6g de carbonate de potassium est chauffé dans 50ml de N,N diméthylformamide durant 4 heures. Le mélange est versé dans l'eau, extrait à l'acétate d'éthyle. La phase organique est lavée, séchée, recristallisée pour obtenir le produit annoncé dont le point de fusion est de 172°C, composé n°32.

### Exemple V : Préparation de 2-(4-chlorobenzylidène)-3,5,5-triméthyl 1-(1H-1,2,4-triazol-1-yl-méthyl)1-cyclopentanol

Un mélange de 2,3g d'hydrure de sodium et 120 ml de DMSO est chauffé à 75°C jusqu'à dissolution du solide. 120 ml de THF sont ajoutés et la solution est refroidie à -5°C. 16 g d'iodure de triméthylsulfonium dans 50 ml de DMSO sont ajoutés en maintenant la température inférieure à 0°C. 15,8 g de 2-(4-chlorobenzylidène) 3,5,5-triméthyl cyclopentanone dissous dans 20 ml de THF sont ajoutés et la solution est laissée à température ambiante.

Une solution de triazolyl sodium est préparée à partir de 5,3 g de 1H 1,2,4-triazole, 2,3 g d'hydrure de sodium dans 100 ml de DMSO est ajoutée puis le mélange est chauffé à 130°C peandant 2 heures. La solution est lavée à l'eau, extraite à l'acétate d'éthyle, séchée, purifiée sur silice. On obtient 2,4 g d'un solide jaune. P.F. = 148°C.

La 2-(4-chloro-benzylidène) 3,5,5-triméthyl cyclopentanone est obtenue de la manière suivante :
A 2,7 g de magnésium dans 50 ml d'éther sont ajoutés 7 ml d'iodure de méthyle. Lorsque l'organomagnésien est formé, la solution est refroidie à -5°C et 1g d'iodure cuivreux sont ajoutés. 10 g de 5,5-diméthyl 2-cyclopenténone dans 30 ml d'éther sont ajoutés en maintenant la température inférieure à 0°C. 14 g de chlorobenzaldehyde dans l'éther sont ensuite ajoutés. 50 ml d'acide chlorhydrique concentré sont ensuite ajoutés puis 50 ml d'eau. La phase aqueuse est ensuite extraite à l'éther. La phase organique est lavée, séchée, purifiée sur colonne de silice. 15,8 g d'un produit huileux sont obtenus. La 5,5-diméthyl 2-cyclopenténone est obtenue de manière connue.

### Exemple VI: Préparation de 2-(4-chlorobenzylidène) 5,5-diallyl 1-(1H-1,2,4-triazol-1-yl-méthyl)-1-cyclopentanol

Un mélange de 2,7 d'hydrure de sodium et 120 ml de DMSO est chauffé à 75°C jusqu'à dissolution. 120 ml de THF sont ajoutés et la solution est refroidie à -5°C. 12,6 g d'iodure de triméthylsulfonium dans 50 ml de DMSO sont ajoutés en maintenant la température inférieure à 0°C. 16,5 g de 2-(4-chlorobenzylidène) 5,5-diallylcyclopentanone dans 20 ml de THF sont ajoutés et la solution est laissée à température ambiante.

Une solution de triazolyl sodium préparée à partir de 8,3 g de 1H 1,2,4-triazole, 4,8 g d'hydrure de sodium dans 100 ml de DMSO est ajoutée puis le mélange est chauffé à 80°C pendant 2 heures. La solution est lavée à l'eau, extraite à l'acétate d'éthyle, séchée, sur colonne de silice. On obtient 6,2 g d'un solide jaune. P.F. = 128°C. Composé n° 33.

La 2-(4-chloro-benzylidène) 5,5-diallyl cyclopentanone est obtenue de la façon suivante :
2,5 g d'hydrure de sodium sont lavés avec 50 ml d'heptane. 100 ml de toluène et 6,7 ml d'alcool tertamylique sont ajoutés et chauffés à 50°C. Lorsque le dégagement d'hydrogène est arrêté, 15 g de 2-(4-chlorobenzylidène) 5-allylcyclopentanone et 8,1 ml de chlorure d'allyle sont ajoutés. La solution est chauffée à reflux, refroidie, lavée à l'eau. La phase organique est séchée. On obtient après évaporation 16,5 g d'un produit liquide. La 5-allylcyclopentanone est obtenue selon W.L. Howard N.B. Lorette Org. Synth. 42, 34.

De la même façon a été obtenu la 2-(4-fluorobenzilidène) 5,5-diallyl 1-(1H-1,2,4-triazol-1-yl-méthyl)-1-cyclopentanol. P.F. 112°C. Composé n° 34.

### Exemple VII : Préparation du 2-(4-chlorobenzylidène) 5,5-diméthyl 1-(1H-1,2,4-triazol-1-ylméthyl)-1-méthoxy cyclopentanol

4,2 g de potasse en poudre et 5,2 g de 2-(4-chlorobenzylidène) 5,5-diméthyl 1-(1H 1,2,4-triazolméthyl) cyclopentane-1-ol dans 32 ml de DMSO sont agités à température ambiante. 2 ml d'iodure de méthyle sont ajoutés et le mélange agité. On verse la solution dans l'eau, extrait à l'acétate d'éthyle. La phase organique est à nouveau lavée à l'eau et sèchée. Après purification sur colonne de silice 4,9 g de produit pur sont obtenus.
P.F. = 84°C.

Le 2-(4-chlorobenzylidène) 5,5 -diméthyl 1-(1H 1,2,4-triazolméthyl) cyclopentane-1-ol est obtenu de la façon suivante :
A un mélange de 10g de 2,2-diméthyl cyclopentanone et 13,8g de 4-chlorobenzaldehyde dans 100ml d'éthanol à 0°C, est ajouté 100ml d'une solution aqueuse de soude à 10%. Aprés 10 minutes une épaisse bouillie était filtrée et le solide lavé puis séché. On obtient 12,5g de 2,2-diméthyl 5-(4-chloro benzylidène) 1-cyclopentanone de point de fusion 120°C.

Ce composé dissout dans 50ml de THF était ajouté à une solution formée de la facon suivante:
1,9 g d'hydrure de sodium (dispersion à 80% dans l'huile minérale) dans 50ml de DMSO anhydre est chauffée à 80°C jusqu'à dissolution totale du solide. Puis la solution est diluée avec 100ml de THF puis refroidi à -10°C. On ajoute au mélange en dix minutes une solution de 11,5g de triméthylsulfonium iodure dans 80ml de diméthylsulfoxide et le mélange était agité pendant 15 minutes à -10°C. Une solution de 11,8g de 2,2-diméthyl 4-chloro 5-(4-chloro benzylidène) 1-cyclopentanone était ensuite ajouté dans 100ml de THF. Le mélange ainsi produit est laissé à température ambiante puis versé dans l'eau et extrait à l'éther, lavé à l'eau, séché, distillé. On obtient le 7-(4-chlorobenzylidène) 4,4-diméthyl 1-oxaspiro [2,4] heptane directement utilisé pour l'étape suivante. Un mélange de 5g du produit avec 2,8g de 1,2,4-triazole et 11g de carbonate de potassium est chauffé dans 40ml de N,N diméthylformamide durant 4 heures. Le mélange est versé dans l'eau, extrait à l'acétate d'éthyle. La phase organique est lavée, séchée, recristallisée pour obtenir le produit annoncé dont le point de fusion est de 154°C. Composé n° 1.

A titre indicatif on donne ci-aprés les points de fusion des composés suivants:
2,2-diméthyl 4-chloro 5-(4-fluoro benzylidène) 1-cyclopentanone de point de fusion 69°C.
2,2-diméthyl 4-chloro 5-(4-chloro benzylidène) 1-cyclopentanone de point de fusion 68°C.
2,2-diméthyl 4-chloro 5-(2,4-dichloro benzylidène) 1-cyclopentanone de point de fusion 68°C.
2,2-diméthyl 5-(4-fluoro benzylidène) 1-cyclopentanone de point de fusion 69°C.
2,2-diméthyl4-chloro 5-(4-chloro benzylidène) 1-cyclopentanone de point de fusion 120°C.
2,2-diméthyl 5-(4-trifluorométhyl benzylidène) 1-cyclopentanone de point de fusion 107°C.
2,2-diméthyl 5-(2,4-dichloro benzylidène) 1-cyclopentanone qui est une huile.
2,2-diméthyl 4-chloro 5-(4-chloro benzylidène) 1-cyclopentanone de point de fusion 60°C.
2-(4-chloro benzylidène) 1-cyclopentanone de point de fusion 63°C.
2-benzylidène 1-cyclopentanone qui est une huile.
2-(4-bromobenzylidène) 1-cyclopentanone de point de fusion 97°C.
2,2-diméthyl 5-benzylidène 1-cyclopentanone qui est une huile.
2,2-diméthyl 5-(3,4-dichlorobenzylidène) 1-cyclopentanone qui est une huile.
2-(3,4-dichlorobenzylidène) 1-cyclopentanone qui est une huile.
2-méthyl 5-(4-chlorobenzylidène) 1-cyclopentanone de point de fusion 107°C.
2-méthyl 5-(2-chlorobenzylidène) 1-cyclopentanone qui est une huile.
2,2-diméthyl 5-(2-chloro benzylidène) 1-cyclopentanone qui est une huile.
2-phénylbenzylidène 1-cyclopentanone de point de fusion 146°C.
2,2-diméthyl 5-phénylbenzylidène 1-cyclopentanone de point de fusion 120°C.
2-éthyl 2-méthyl 5-(4-chloro benzylidène) 1-cyclopentanone de point de fusion 85°C.
2-allyl 5-(4-chloro benzylidène) 1-cyclopentanone de point de fusion 60°C.
2,2-diallyl 5-(4-chloro benzylidène) 1-cyclopentanone qui est une huile.
2,2,4-triméthyl 5-(4-chloro benzylidène) 1-cyclopentanone qui est une huile.
2-méthyl-2-méthoxyméthyl 5-(4-chloro benzylidène) 1-cyclopentanone qui est une huile.
2-allyl 5-(4-fluorobenzylidène) 1-cyclopentanone de point de fusion 111°C.
2,2-diallyl 5-(4-fluorobenzylidène) 1-cyclopentanone qui est une huile.
2,2-diéthyl 5-(4-chloro benzylidène) 1-cyclopentanone qui est une huile.
2,2-diéthyl 5-(4-fluorobenzylidène) 1-cyclopentanone qui est une huile.
2-(4-cyanobenzylidène) 1-cyclopentanone qui est une huile.
2,2-diméthyl 5-(4-cyanobenzylidène) 1-cyclopentanone qui est une huile.
2,2-diméthyl 4-éthyl 5-(4-chloro benzylidène) 1-cyclopentanone qui est une huile .
2-(4-p-chlorophénoxy benzylidène) 1-cyclopentanone de point de fusion 92°C.
2,2-diméthyl 5-(4-p-chlorophénoxybenzylidène) 1-cyclopentanone qui est une huile.
2,2-diméthyl 4-isopropyl 5-(4-chloro benzylidène) 1-cyclopentanone qui est une huile.
2,2-diméthyl 5-(4-chloro benzylidène) 1-cyclopentanone de point de fusion 60°C.
2,2-diméthyl 5-(2,4-dichloro benzylidène) 1-cyclopentanone de point de fusion 68°C.
2,2-diméthyl 5-(4-fluoro benzylidène) 1-cyclopentanone de point de fusion 69°C.

### Exemple VIII : Préparation des 2-(4-chlorobenzylidène) 6-méthyl 1-(1H-1,2,4-triazol-1-ylméthyl)-1-cyclohexanols

A un mélange de 22,4 g de 2-méthyl cyclohexanone et de 28,1 g de para chlorobenzaldéhyde dans 250 ml d'éthanol à 0°C est ajouté 100 ml d'une solution aqueuse à 10 % de soude. Après 15 h d'agitation, le milieu est dilué à l'eau et extrait à l'acétate d'éthyle.

La phase organique séchée sur Na₂SO₄ est concentrée et le résidu chromatographié sur silice avec un éluant heptane/acétate d'éthyle 90/10. Une huile jaune est obtenue. Celle-ci est cristallisée dans le pentane et donne une poudre jaune pâle (17,1 g) de 2-(4-chlorobenzylidène) 6-méthylcyclohexanone dont le point de fusion est de 59°C.

3,8 g de ce composé en solution dans le THF anhydre (33 ml) sont ajoutés à un milieu réactionnel à -5°C préparé de la manière suivante :
0,8 g d'hydrure de sodium (dispersion à 60 %) sont chauffés à 70°C dans 26 ml de DMSO sec, puis on ajoute 33 ml de THF anhydre. Le mélange refroidit à -5°C est traité par de l'iodure de triméthylsulfonium (3,9 g) en solution dans le DMSO sec (20 ml).

Après 1 heure à -5° le 8-(4-chlorobenzylidène) 4-méthyl 1-oxaspiro [2,5] octane ainsi formé est traité par du triazole (2,2 g) et du triazolylsodium (0,28 g).

Le milieu est chauffé à 93° pendant 1h 30 après distillation de 30 ml de THF et addition de DMF (50 ml).

Après dilution à l'eau (1l), les produits sont extraits au dichlorométhane et le résidu obtenu par évaporation est purifié par chromatographie (éluant heptane/acétate d'éthyle 1:1 ).

On obtient d'abord le diastéréoisomère majeur qui possède le substituant méthyle et l'hydroxyle en position relative cis et fondant à 139° (composé n° 35), puis le diastéréoisomère mineur qui possède le substituant méthyle et l'hydroxyle en position relative trans sous forme d'un miel (composé n° 36).

La stéréochimie est attribuée par résonance magnétique nucléaire selon des techniques bien connues de l'homme de l'art.

### Exemple n° IX : Préparation de 2-(4-chlorobenzylidène) -6,6-diméthyl 1-(1H-1,2,4-triazol-1-yl-méthyl)-1-cyclohexanol

A un milieu réactionnel préparé à partir de toluène (25 ml) d'alcool tertioamylique (3,6 ml) et d'hydrure de sodium (1,35 g de dispersion dans l'huile à 60 %) et chauffé à 45°C, on ajoute successivement la 2-(4-chlorobenzylidène) 6-méthyl cyclohexanone préparée ci-dessus (7,0 g) puis de l'iodure de méthyle (5,1 g). Le mélange est porté à reflux (1h) versé dans l'eau et extrait à l'acétate d'éthyle. Après sèchage et concentration, le résidu est recristallisé dans le méthanol pour donner la 2-(4-chlorobenzylidène) 6,6-diméthyl cyclohexanone (4,7 g) fondant à 92°C.

La procédure de l'exemple I est ensuite appliquée à 4 g de cette cétone pour conduire après chromatographie au produit attendu fondant à 179°C (composé n° 37).

De la même facon ont été obtenus les composés suivants:
2-(4-fluorobenzylidène) -6,6-diméthyl 1-(1H-1,2,4-triazol-1-yl-méthyl)-1-cyclohexanol. Isomère A. Composé n°38. P.F 154°C.
2-(4-chlorobenzylidène) 1-(1H-1,2,4-triazol-1-yl-méthyl)-1-cyclohexanol. Isomère A. Composé n°39. P.F 117°C.
2-(4-chlorobenzylidène) -6-cyclohéxyl 1-(1H-1,2,4-triazol-1-yl-méthyl)-1-cyclohexanol. Isomère A. Composé n°40. P.F 206°C.
2-(4-chlorobenzylidène) -6-cyclohéxyl 1-(1H-1,2,4-triazol-1-yl-méthyl)-1-cyclohexanol. Isomère B. Composé n°41. P.F 197°C.
2-(4-chlorobenzylidène) -6-t-butyl 1-(1H-1,2,4-triazol-1-yl-méthyl)-1-cyclohexanol. Isomère A. Composé n°42. P.F 144°C.
2-(4-chlorobenzylidène) -6-sec-butyl 1-(1H-1,2,4-triazol-1-yl-méthyl)-1-cyclohexanol. Isomères A+B. Composé n°43. P.F 171°C.
2-(4-chlorobenzylidène) -6-sec-butyl 1-(1H-1,2,4-triazol-1-yl-méthyl)-1-cyclohexanol. Isomères C+D. Composé n°44. P.F 148°C.
2-(4-chlorobenzylidène) -6-méthyl,- 6-phényl 1-(1H-1,2,4-triazol-1-yl-méthyl)-1-cyclohexanol. Isomère A. Composé n°45. P.F 177°C.
2-(4-chlorobenzylidène) -6-méthyl,-6-phényl 1-(1H-1,2,4-triazol-1-yl-méthyl)-1-cyclohexanol. Isomère B. Composé n°46. P.F 133°C.
2-(4-chlorobenzylidène) -6-méthyl, -6-éthyl 1-(1H-1,2,4-triazol-1-yl-méthyl)-1-cyclohexanol. Isomères A+B. Composé n°47. P.F 175°C.
2-(4-chlorobenzylidène) -6-méthyl,-6-isopropyl 1-(1H-1,2,4-triazol-1-yl-méthyl)-1-cyclohexanol. Isomère A. Composé n°48. P.F 154°C.
2-(4-chlorobenzylidène) -6-méthyl,-6-isobutyl 1-(1H-1,2,4-triazol-1-yl-méthyl)-1-cyclohexanol. Isomère A. Composé n°49. P.F 163°C.
2-(4-chlorobenzylidène) -6-méthyl,-6-isobutyl 1-(1H-1,2,4-triazol-1-yl-méthyl)-1-cyclohexanol. Isomère B. Composé n°50. P.F 184°C.
2-(4-chlorobenzylidène) 1-(1H-1,2,4-triazol-1-yl-méthyl)-1-cycloheptanol. Composé n°54. P.F 104°C.
2-(4-chlorobenzylidène) -7,7-diméthyl 1-(1H-1,2,4-triazol-1-yl-méthyl)-1-cycloheptanol. Composé n°54. P.F 143°C.
2-(4-fluorobenzylidène) 1-(1H-1,2,4-triazol-1-yl-méthyl)-1-cycloheptanol. Composé n°56. P.F 91°C.
2-(4-chlorobenzylidène) -7-méthyl 1-(1H-1,2,4-triazol-1-yl-méthyl)-1-cycloheptanol. Composé n°56. P.F 166°C.

A titre indicatif on donne ci-aprés les caractéristiques de quelques cycloalcanones de départ. Le lecteur notera que des méthodes génerales de préparation de ces composés sont indiqués dans la littérature. On pourra se référer utilement aux documents suivants:
DOS 2245518
D. A. WHITING J., Chem. Soc (1971), 3396
G. Le GUILLANTON (1969), Bull. Soc . Chim, 2871
PAL PERJESI et al, Chem. Ber.(1987), 120, 1449.
J.M. CONIA et al, Bull. Soc. Chim. (1970), 2972.
2-(4-fluorobenzylidène) 1-cyclohexanone de point d'ébulition à 0,02mm de Hg 140°C.
2-(4-chlorobenzylidène) 1-cyclohexanone de point de fusion 54°C.
2-méthyl 6-(4-chlorobenzylidène) 1-cyclohexanone de point de fusion 59°C.
2,2-diméthyl 6-(4-chloro benzylidène) 1-cyclohexanone de point de fusion 92°C.
2,2-diméthyl 6-(4-fluorobenzylidène) 1-cyclohexanone de point de fusion 51°C.
2-cyclohexyl 6-(4-chlorobenzylidène) 1-cyclohexanone de point de fusion 97°C.
2-t-butyl 6-(4-chlorobenzylidène) 1-cyclohexanone de point d'ébbulition à 0,06mm de Hg 180°C.
2-sec-butyl 6-(4-chlorobenzylidène) 1-cyclohexanone de point de fusion 46°C.
2-phényl 6-(4-chlorobenzylidène) 1-cyclohexanone de point de fusion 121°C.
2-méthyl 2-phényl 6-(4-chlorobenzylidène) 1-cyclohexanone de point de fusion 77°C.
1,1-diméthyl 3-(4-chloro benzylidène) 2-tétralone qui est une huile.
2-méthyl 2-éthyl 6-(4-chlorobenzylidène) 1-cyclohexanone de point de fusion 70°C.
2-méthyl 2-isobutyl 6-(4-chlorobenzylidène) 1-cyclohexanone qui est une huile.
2-méthyl 2-isopropyl 6-(4-chlorobenzylidène) 1-cyclohexanone qui est une huile.
6-(4-chloro benzylidène) spiro[4,5] décan-5-one qui est une huile.
2-(4-fluorobenzylidène) 1-cycloheptanone de point de fusion 62°C.
2-(4-chlorobenzylidène) 1-cycloheptanone de point de fusion 75°C.
2-méthyl 7-(4-chlorobenzylidène) 1-cycloheptanone de point de fusion 74°C.
2,2-diméthyl 7-(4-chlorobenzylidène) 1-cycloheptanone de point de fusion 63°C.
2,2-diméthyl 7-(4-chlorobenzylidène) 1-cyclohexanone de point de fusion 63°C.
- Les exemples suivants illustrent les applications fongicides des composés selon l'invention.

Dans ces exemples, les pulvérisations de solutions ou suspensions de matières actives sont effectuées dans des conditions telles que la pulvérisation d'une solution ou suspension de concentration égale à 1 g/l correspond en moyenne à l'application d'environ 2 microgrammes de matière active par cm2 de feuille de la plante.

Dans les conditions des exemples X à XVI, les composés illustrés n'ont pas présenté de phytotoxicité.

Dans ces exemples on considère qu'un produit exerce une protection totale vis à vis d'une maladie fongique lorsque la protection est d'au moins 95 % ; la protection est considérée comme bonne lorsqu'elle est d'au moins 80 % (mais inférieure à 95 %), comme assez bonne lorsqu'elle est d'au moins 70 % (mais inférieure à 80 %), comme moyenne lorsqu'elle est d'au moins 50 % (mais inférieure à 70 %).

Dans le présent exposé les pourcentages sont, sauf indication contraire et sauf ceux concernant les rendements, des pourcentages pondéraux. Dans le cas où les pourcentages sont exprimés par rapport à la stoechiométrie, il s'agit de pourcentages molaires. En ce qui concerne les concentrations, certaines d'entre elles sont exprimées en ppm (partie par million) ce qui correspond à des mg/l.

Les essais ont été effectués à des doses de 1 g/l ou 0,3 g/l sans que cela préjuge sur la valeur des produits eux-mêmes.

### Exemple X - Test in vivo sur Botrytis cinerea sur feuille excisée de tomate

On prépare par broyage fin une émulsion aqueuse de la matière active à tester ayant la composition suivante :
- matière active à tester : 60 mg
- Tween 80 (agent tensio actif) constitué d'un oléate de dérivé polyoxyéthylèné du sorbitan) dilué à 10 % dans l'eau : 0,3 ml
- on complète à 60 ml d'eau.

Cette émulsion aqueuse est ensuite diluée par de l'eau pour obtenir la concentration désirée.

Des tomates cultivées en serre (variété Marmande) agées de 30 à 40 jours sont traitées par pulvérisation avec des émulsions aqueuses (appelées bouillies) telles que définies ci-dessus et à diverses concentrations du composé à tester. L'essai est répété deux fois avec chaque concentration.

Après 24 ou 48 heures, les feuilles sont coupées et mises dans 2 boites de Pétri (diamètre 14 cm) dont le fond a été préalablement garni d'un disque de papier filtre humide (5 folioles par boite).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (100.000 unités/cm3).

Le contrôle est fait à 3 et 6 jours après la contamination par comparaison avec un témoin non traité.

Dans ces conditions on observe aprés 6 jours, à la dose de 1 g/l, une protection bonne ou totale avec les composés 1, 3, et à la dose de 0,3 g/l une protection bonne ou totale avec les composés 2, 15, 16, 35, 38, 39.

### Exemple XI - Test in vivo sur Erysiphe graminis sur orge (oïdium de l'orge)

De l'orge, en godets, semée sur un substrat tourbe terre pouzzolane 50/50, est traitée au stade 10 cm de hauteur par pulvérisation d'une émulsion aqueuse (appelée bouillie), comme indiquée ci avant, de concentration indiquée ci-après. L'essai est répété deux fois. Au bout de 24 heures, on saupoudre les plants d'orge avec des spores d'Erysiphe graminis, le saupoudrage étant effectué à l'aide de plants malades.

La lecture se fait 8 à 14 jours après la contamination.

Dans ces conditions, on observe les résultats suivants :
A la dose de 1 g/l, protection bonne ou totale avec les composés 1, 2, 3, 14, 26 et à 0,3 g/l avec les composés 4, 11, 38, 53, 55.

A la dose de 1 g/l, protection assez bonne ou moyenne avec les composés 10, 25, 33 et à 0,3 g/l avec les composés 8, 15, 22, 29, 39.

### Exemple XII - Test in vivo sur "Puccina recondita" responsable de la rouille du blé

Du blé, en godets, semé sur un substrat tourbe terre pouzzolane 50/50, est traité au stade 10 cm de hauteur par pulvérisation avec des émulsions aqueuses (appelées bouillies) de même composition que celle décrite à l'exemple IV et à diverses concentrations du composé à tester. L'essai est répété deux fois avec chaque concentration.

Au bout de 24 heures, une suspension aqueuse de spores (50000 sp/cm3) est pulvérisée sur le blé ; cette suspension a été obtenue à partir de plants contaminés. On place ensuite le blé pendant 48 heures en cellule d'incubation à environ 18°C et à 100 % d'humidité relative.

Au bout de ces 2 jours, l'humidité relative est ramenée à 60 %. Le contrôle de l'état des plants se fait entre le 11ème et le 15ème jour après la contamination par comparaison avec le témoin non traité.

A la dose de 1g/l, protection bonne ou totale avec les composés 1, 2, 3, 10, 14, 25, 26, 27, 33, et à 0,3 g/l avec les composés 4, 7, 9, 13, 14, 15, 18, 19, 28, 34, 35, 37, 38, 43, 47, 53.

A la dose de 2 g/l, protection assez bonne ou moyenne avec le composé 5 et à 0,3 g/l avec les composés 8, 10, 11, 20, 22, 42, 44, 49.

### Exemple XIII - Test in vivo sur "Piricularia oryzae" responsable de la Piriculariose du riz (Rice Blast)

Du riz, en godets, semé dans un mélange 50/50 de tourbe enrichie et de pouzzolane, est traité au stade 10 cm de hauteur par pulvérisation d'une émulsion aqueuse (appelée bouillie) ci-dessus définie de concentration indiquée ci-après. L'essai est répété deux fois. Au bout de 48 heures, on traite par application sur les feuilles, par une suspension de spores obtenues en culture pure.

La lecture se fait 8 jours après la contamination. Dans ces conditions, on observe les résultats suivants :
A la dose de 1g/l, protection bonne ou totale avec les composés 1, 2, 4, 25, 28, et à
0,3 g/l avec les composés 7, 9, 11, 12, 13, 14, 22, 29, 30, 33, 34, 38, 39, 43, 44, 47.

A la dose de 1 g/l, protection assez bonne ou moyenne avec les composés 5, 26, 27, et à 0,3 g/l avec les composés 15, 16, 18, 23, 31, 32, 42, 48, 49.

### Exemple XIV - Test in vivo sur "Puccinia recondita par application en traitement de semences

Des graines de blé variété Talent sont traitées avec la bouillie de l'exemple précédent à la dose de 75 g/q, semées dans un substrat composé d'un mélange tourbe terre pouzzolane 50/50. 15 jours aprés le semis les plantules sont inoculées par Puccinia recondita, selon le protocole décrit à l'exemple XII. La lecture des résultats est faite 30 jours et 45 jours aprés le semis.

Aux doses indiquées avec les composés n°1, 2,3, 4, 13, 28, 29, 30, 31, 32, 35, et 37, le pourcentage d'infection est nul à 30 jours aprés le semis, les plantules issues de graines non traitées étant contaminées à 100% .

### Exemple XV Test in vivo sur Fusarium roseum par application en traitement de semences

Des graines de blé variété Talent, naturellement contaminées par Fusarium roseum sont traitées avec une bouillie ci avant définie à l'exemple X aux doses de 10, 25, 50, 100 g pour
100 kg de graines. Parmi 50 g de graines traitées, 200 graines sont déposées sur un milieu contenant de la gelose et du malt dans les concentrations respectives de 2 % et 1 %. Les graines sont conservées pendant 10 jours à 20°C. Le contrôle de l'état des graines se fait par comparaison avec le témoin non traité où se sont développées des colonies de Fusarium roseum.

Aux doses indiquées, on observe une protection bonne ou totale avec les composés 1, 2, 3, 4, 13, 15, 28, 29, 30, 31, 32, 35, 37, 52, 54.

L'exemple ci dessous illustre l'absence de phytotoxicité de composés selon l'invention vis à vis de la croissance des graines.

### Exemple XVI

Des grains de blé variété talent sont traitées avec une bouillie aux doses de 2.5, 10, 25, 50, 100, 200, 400g/ q.

Les graines sont déposées sur un papier filtre imbibé d'eau. Aprés 15 jours d'incubation à 25°C les longueurs des coléoptiles et des premiéres feuilles sont mesurées pour les composés n°1, 13, 15, 35, et 37.

Les résultats sont présentés dans le tableau ci-dessous :

| dose g/q | longueur (cm) coléoptiles et premières feuilles |
|---|---|
| 0 | 13 |
| 12 | 13 |
| 25 | 13 |
| 50 | 13 |
| 100 | 12 |
| 200 | 13 |
| 400 | 12 |

Chaque valeur indiquée ci dessus est la moyenne de quarante plantules.

## Revendications

1. Benzylidène azolylméthylcycloalcanes ou alcènes de formule : 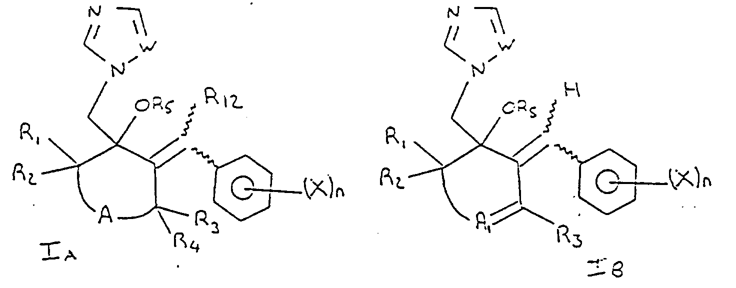 dans lesquelles
A est -CR₆ R₇- ou -CR₆ R₇ CR₈ R₉- ou - CR₆ R₇ CR₈ R₉ CR₁₀ R₁₁-
A1 est CR₇=, - CR₆ R₇- CR₉=, -CR₆ R₇-CR₈ R₉-CR₁₁=,
X est un groupe cyano ou nitro, ou un groupe C1-C4 alkyle ou C1-C4 alkoxy éventuellement halogénés ou un atome d'halogène,
n est un nombre entier positif ou nul, inférieur à 6, les groupements X pouvant être identiques ou différents lorsque n est plus grand que 1,
W représente un groupe trivalent constitué soit d'un groupe = CH-, soit d'un atome d'azote = N-,
R1, R2, identiques ou différents, représentent l'atome d'hydrogène ou un radical C1-C4 alkyle (éventuellement substitué par un ou plusieurs atomes ou radicaux choisis parmi les atomes d'halogènes, les radicaux C1-C4 alkoxy, mono ou poly halo C1-C4 alkoxy, C₂-C₄ alcényle, C₂-C₄ alcynyle, mono ou poly halo C₂-C₄ alcényle, mono ou poly halo C₂-C₄ alcynyle), C1-C4 alkoxy (éventuellement substitué par un ou plusieurs atomes ou radicaux choisis parmi les atomes d'halogènes, les radicaux C1-C4 alkoxy, mono ou polyhalo C1-C4 alkoxy, C₂-C₄ alcényle, C₂-C₄ alcynyle, mono ou polyhalo C₂-C₄ alcényle, mono ou polyhalo C₂-C₄ alcynyle), les radicaux C₃-C₇ cycloalkyle , C₆-C₁₀ aryle, C₇-C₁₁ aralkyle, ces divers radicaux pouvant être éventuellement substitués par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy, R₁, R₂ ensemble peuvent former une chaîne C₂-C₅ hydrocarbonée constituant un cycle avec le carbone auquel R₁, R₂ sont reliés, cette chaîne étant éventuellement substituée comme pour les radicaux C₆-C₁₀ aryle précédemment cités ou R₁, R₂ ensemble peuvent former une chaîne C₂-C₅ hydrocarbonée dioxolane avec le carbone auquel R₁, R₂ sont reliés, cette chaîne étant éventuellement substituée comme pour les radicaux C₆-C₁₀ aryle précédemment cités.
R₃, R₆ à R₁₁, identiques ou différents, représentent l'atome d'hydrogène ou un radical C₁-C₄ alkyle (éventuellement substitué par un ou plusieurs atomes ou radicaux choisis parmi les atomes d'halogènes, les radicaux C₁-C₄ alkoxy, mono ou polyhalo C₁-C₄ alkoxy), les radicaux C₃-C₇ cycloalkyle, C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), ou bien deux radicaux adjacents de la chaîne A forment ensemble, avec les atomes de A auxquels ils sont rattachés, un cycle phényle accolé au cycloalcane,
R₅ représente l'atome d'hydrogène, un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle,) les radicaux C₃-C₇ cycloalkyle , C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), ou R₅ représente un groupe C(=O)-R₁₃, R₁₃ représentant un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle,) les radicaux C₃-C₇ cycloalkyle , C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), C₂-C₄ éthynyle, C₂-C₄ acétynyle, mono ou polyhalo C₂-C₄ éthynyle, mono ou polyhalo C₂-C₄ acétynyle,
R₁₂ a l'une des significations de R₅, à l'exception de C(=O)-R₁₃,
R₄ représente l'atome d'hydrogène, un atome d'halogène, notamment un atome de chlore ou de brome, un radical C₁-C₄ alkyle éventuellement substitué (par un ou plusieurs atomes ou radicaux choisis parmi les atomes d'halogènes, les radicaux C₁-C₄ alkoxy, mono ou polyhalo C₁-C₄ alkoxy, C₂-C₄ alcényle, C₂-C₄ alcynyle, mono ou polyhalo C₂-C₄ alcényle, mono ou polyhalo C₂-C₄ alcynyle,) les radicaux C₃-C₇ cycloalkyle , C₆-C₁₀ aryle, C₇-C₁₁ aralkyle, ces divers radicaux pouvant être éventuellement substitués (par un ou plusieurs atomes ou radicaux choisis parmi les atomes d'halogènes, les radicaux C₁-C₄ alkyle, les radicaux mono ou polyhalo C₁-C₄ alkyle, les radicaux C₁-C₄ alkoxy et les radicaux mono ou polyhalo C₁-C₄ alkoxy), et les formes salifiées acceptables en agriculture de ces composés.

2. Composés selon la revendication 1, caractérisés en ce que n = 1, 2 ou 3.

3. Composés selon la revendication 2, caractérisés en ce que X est un atome d'halogène choisi parmi le chlore, le brome, le fluor.

4. Composés selon la revendication 3, caractérisés en ce que n = 1 ou 2 et X est placé en para lorsque n = 1, et méta-para ou ortho-para lorsque n = 2.

5. Composés selon la revendication 4, caractérisés en ce que n = 1.

6. Composés selon la revendication 3, 4 ou 5, caractérisés en ce que X est l'atome de chlore.

7. Composés selon la revendication 1, caractérisés en ce que W est l'atome d'azote.

8. Composés selon la revendication 1, caractérisés en ce que R₃, R₆, R₉, R₁₀, sont l'atome d'hydrogène.

9. Composés selon la revendication 8, caractérisés en ce que R₄, R₇, R₉, R₁₁, sont l'atome d'hydrogène ou le radical C₁-C₄ alkyle.

10. Composés selon la revendication 1, caractérisés en ce que R₁, R₂, sont choisis parmi les radicaux méthyle ou éthyle ou l'atome d'hydrogène.

11. Composés selon la revendication 1, caractérisés en ce que R₅, est l'atome d'hydrogène.

12. Composés selon la revendication 1, caractérisés en ce que R₁₂, est l'atome d'hydrogène.

13. Composés selon la revendication 1, caractérisés en ce qu'ils répondent à la formule IA.

14. Composés selon la revendication 13, caractérisés en ce que lorsque A est CR₆R₇, R₁, R₂ sont choisis parmi les radicaux méthyle ou éthyle, R₃, R₅ à R₇, R₁₂ sont l'atome d'hydrogène, R₄ est méthyle, éthyle, i-propyle, n-propyle, ou l'atome d'hydrogène.

15. Composés selon la revendication 14, qui est le 2-(4-chlorobenzylidène)5,5-diméthyl 1-(1H-1,2,4-triazol-1-ylméthyl)-1-cyclopentanol, ou le - 2-(4-chlorobenzylidène)-5-méthyl 5-éthyl 1-(1H-1,2,4-triazol-1-ylméthyl)-1-cyclopentanol

16. Composés selon la revendication 13, caractérisés en ce que lorsque A est CR₆R₇ CR₈R₉, R₁, R₂ sont choisis parmi les radicaux méthyle éthyle ou l'atome d'hydrogène, R₃, R₅ à R₉, R₁₂ sont l'atome d'hydrogène, R₄ est le radical méthyle, éthyle, i-propyle, n-propyle, ou l'atome d'hydrogène.

17. Composés selon la revendication 13, qui sont
- 2-(4-chlorobenzylidène)-6-méthyl 1-(1H-1,2,4-triazol-1-ylméthyl)-1-cyclohexanol
- 2-(4-chlorobenzylidène)-6,6-diméthyl 1-(1H-1,2,4-triazol-1-yl-méthyl)
- 2-(4-chlorobenzylidène)-1-(1H-1,2,4-triazol-1-ylméthyl)-1-cyclohexanol.

18. Composition fongicide comprenant 0,5 % à 95 % de composés selon l'une des revendications 1 à 17, en combinaison avec les supports solides ou liquides acceptables en agriculture et/ou ces agents tensio-actifs également acceptables en agriculture.

19. Procédé de traitement des cultures atteintes ou susceptibles d'être atteintes par les maladies fongiques caractérisé en ce que l'on applique sur les feuilles une quantité efficace d'un compose selon l'une des revendications 1 à 17 ou d'une composition selon la revendication 18.

20. Procédé de traitement selon la revendication 19, caractérisé en ce que l'on applique une dose comprise entre 0,002 et 5 kg/ha.

21. Produit de multiplication des plantes cultivés qui a subi un traitement de protection par un des composés selon l'une des revendications 1 à 17 ou une composition selon la revendication 18.

22. Graine selon la revendication 21, ayant subi un traitement de protection à raison de 0,1 à 500 g par quintal.

23. Composés utiles notamment comme intermédiaire dans la préparation des composés selon les revendications 1 à 17, répondant aux formules 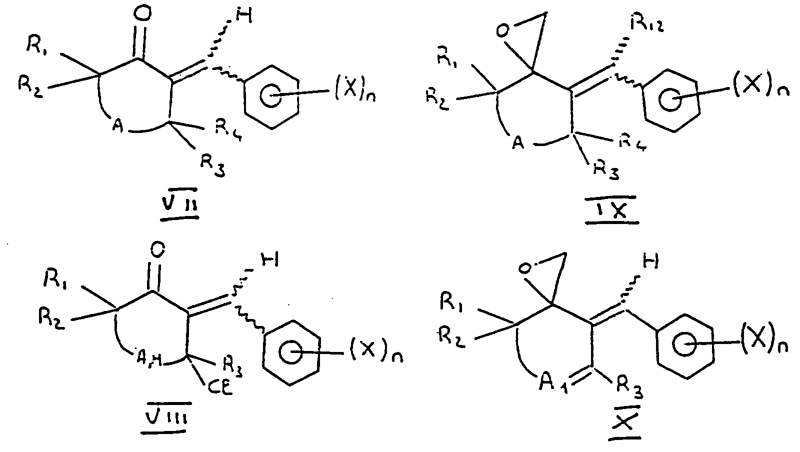 dans lesquelles :
- les substituants n, X, R₁, R₂, A₁, R₄ A, R₃, R₁₂ ont la même signification que dans la revendication 1,
- et le groupe A₁H de la formule VIII correspond aux groupes -CR₇H, ou -CR₆R₇-CR₉H ou - -CR₆R₇-CR₈R₉-CR₁₁H, dans lesquels les substituants R₆, R₇, R₈, R₉, R₁₁, ont la même signification que dans la revendication 1,
sauf les composés suivants :
2-benzylidènecyclopentanone,
2-(4-chlorobenzylidène) cyclopentanone,
2-(4-chlorobenzylidène)-5-benzylcyclopentanone,
2-(4-bromobenzylidène)-5-benzylcyclopentanone,
2-benzylidène-3-méthylcyclopentanone,
2-benzylidène-5-méthyl-cyclopentanone,
2-benzylidène-5-benzylcyclopentanone,
2-benzylidènecyclohexanone,
6-cyclohexyl-2-benzylidènecyclohexanone,
2-benzylidène-5-méthyl-6-cyclohexyl-cyclohexanone,
2-(4-méthoxybenzylidène)-4-méthyl-6-phénylcyclohexanone,
2-benzylidène-6(4-isopropylphényl)cyclohexanone,
2-benzylidène-6-méthyl-cyclohexanone,
2-benzylidène-4-méthyl-cyclohexanone,
2-benzylidènecycloheptanone,
2-benzylidène-6-benzyl-cyclohexanone ;
et à l'exclusion des composés de formule VII, dans laquelle :
- R₁ est un atome d'hydrogène ; et
- R₂ est un atome d'hydrogène ou un radical cyclohexyle ou phényle, ledit radical étant éventuellement substitué par un radical C₁-C₄ alkyle ; et
- R₃ est un atome d'hydrogène ; et
- R₄ est un atome d'hydrogène ou un radical méthyle, éthyle, ou propyle ; et
- A est -CHR₆-, -CHR₆-CHR₈-, -CHR₆-CHR₈-CHR₁₀- ; et
- R₆, R₈, R₁₀ sont choisis parmi un atome d'hydrogène, ou un radical méthyle, éthyle, ou propyle ; et
- n égale 0, 1 ou 2 ; et
- X est un atome d'hydrogène ou d'halogène, un radical C₁-C₄ alkyle ou C₁-C₄ alkoxy, ou un groupe nitro.

24. Procédé de préparation de composés de formule IA, dans laquelle R₁, R₂, R₃, R₁₂, X, n, W, A ont la même signification que dans la revendication 1, R₄ est différent de l'atome d'halogène, R₅ est l'atome d'hydrogène, caractérisé en ce que un composé de formule VII est mis à réagir avec une ylure de sulfonium pour conduire aux oxirannes de formule IX, puis ce dernier est mis à réagir avec une imidazole ou triazole non substitué en présence d'une base.

25. Procédé de préparation de composés de formule IA, dans laquelle R₁, R₂, R₃, R₁₂, X, n, W, A ont la même signification que dans la revendication 1, R₄ est l'atome d'halogène, R₅ est l'atome d'hydrogène, par halogénation allylique des composés de formule (I) où R₄, R₅ sont l'atome d'hydrogène.

26. Procédé de préparation de composés de formule IA, dans laquelle R₁, R₂, R₃, R₁₂, X, n, W, A ont la même signification que dans la revendication 1, R₄ est différent de l'atome d'halogène, R₅ est différent de l'atome d'hydrogène, par estérification ou éthérification des composés de formule IA obtenus selon la revendication 24.

27. Procédé de préparation de composés de formule IA, dans laquelle R₁, R₂, R₃, R₁₂, X, n, W, A ont la même signification que dans la revendication 1, R₄ est l'atome d'halogène, R₅ est différent de l'atome d'hydrogène, sont obtenus dans une première étape par estérification ou éthérification selon la revendication 26, d'un composé de formule I dans laquelle R₄, R₅ sont l'atome d'hydrogène suivi d'une halogénation allylique.

28. Procédé de préparation de composés de formule IB, dans laquelle R₁, R₂, A, n, X ont la même signification que dans la revendication 1, R₅ est l'atome d'halogène, caractérisé en ce que l'on fait réagir un composé de formule VIII avec une ylure de sulfonium pour obtenir un oxiranne de formule X suivi d'une réaction avec un imidazole ou triazole en présence d'une base suivi éventuellement d'une estérification ou éthérification pour obtenir un composé où R₅ est l'atome d'hydrogène.

## Claims

1. (Benzylidene)-azolylmethylcycloalkanes or -alkenes of formula: 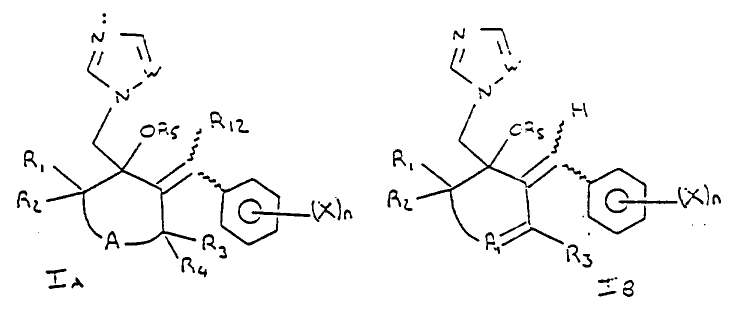 in which
A is -CR₆R₇- or -CR₆R₇CR₈R₉- or -CR₆R₇CR₈R₉CR₁₀R₁₁-,
A₁ is CR₇=, -CR₆R₇-CR₉= or -CR₆R₇-CR₈R₉-CR₁₁=,
X is a cyano or nitro group, or a C1-C4 alkyl or C1-C4 alkoxy group, optionally halogenated, or a halogen atom,
n is a positive integer or zero, less than 6, it being possible for the groups X to be identical or different when n is greater than 1,
W denotes a trivalent group consisting of either a =CH- group or a nitrogen atom =N-,
R1 and R2, which may be identical or different, denote a hydrogen atom or a C1-C4 alkyl radical, (optionally substituted with one or more atoms or radicals selected from halogen atoms and C1-C4 alkoxy, mono- or polyhalo(C1-C4 alkoxy), C₂-C₄ alkenyl, C₂-C₄ alkynyl, mono- or polyhalo(C₂-C₄ alkenyl) and mono- or polyhalo(C₂-C₄ alkynyl) radicals), C1-C4 alkoxy radical, (optionally substituted with one or more atoms or radicals selected from halogen atoms and C1-C4 alkoxy, mono- or polyhalo(C1-C4 alkoxy), C₂-C₄ alkenyl, C₂-C₄ alkynyl, mono- or polyhalo(C₂-C₄ alkenyl) and mono- or polyhalo(C₂-C₄ alkynyl) radicals), or C₃-C₇ cycloalkyl, C₆-C₁₀ aryl or C₇-C₁₁ aralkyl radicals, it being possible for these various radicals to be optionally substituted with one or more atoms or radicals selected from halogen atoms, C₁-C₄ alkyl radicals, mono- or polyhalo(C₁-C₄ alkyl) radicals, C₁-C₄ alkoxy radicals and mono- or polyhalo(C₁-C₄ alkoxy) radicals, R₁ and R₂ together can form a C₂-C₅ hydrocarbon chain making a ring with the carbon to which R₁ and R₂ are attached, this chain optionally being substituted as for the C₆-C₁₀ aryl radicals mentioned above, or R₁ and R₂ together can form a C₂-C₅ dioxolane hydrocarbon chain with the carbon to which R₁ and R₂ are attached, this chain optionally being substituted as for the C₆-C₁₀ aryl radicals mentioned above,
R₃ and R₆ to R₁₁, which may be identical or different, denote a hydrogen atom or a C₁-C₄ alkyl radical, (optionally substituted with one or more atoms or radicals selected from halogen atoms and C₁-C₄ alkoxy and mono- or polyhalo(C₁-C₄ alkoxy) radicals), or C₃-C₇ cycloalkyl, C₆-C₁₀ aryl or C₇-C₁₁ aralkyl radicals, it being possible for these various radicals to be optionally substituted (with one or more atoms or radicals selected from halogen atoms and C₁-C₄ alkyl radicals, mono- or polyhalo(C₁-C₄ alkyl) radicals, C₁-C₄ alkoxy radicals and mono- or polyhalo(C₁-C₄ alkoxy) radicals), or alternatively two adjacent radicals of the chain A, together with the atoms of A to which they are attached, form a benzene ring fused to the cycloalkane,
R₅ denotes a hydrogen atom, a C₁-C₄ alkyl radical, optionally substituted (with one or more atoms or radicals selected from halogen atoms and C₁-C₄ alkoxy, mono- or polyhalo(C₁-C₄ alkoxy), C₂-C₄ alkenyl, C₂-C₄ alkynyl, mono- or polyhalo(C₂-C₄ alkenyl) and mono- or polyhalo(C₂-C₄ alkynyl) radicals), or C₃-C₇ cycloalkyl, C₆-C₁₀ aryl or C₇-C₁₁ aralkyl radicals, it being possible for these various radicals to be optionally substituted (with one or more atoms or radicals selected from halogen atoms and C₁-C₄ alkyl radicals, mono- or polyhalo(C₁-C₄ alkyl) radicals, C₁-C₄ alkoxy radicals and mono- or polyhalo(C₁-C₄ alkoxy) radicals), or R₅ denotes a group C(=O)-R₁₃, R₁₃ denoting a C₁-C₄ alkyl radical, optionally substituted (with one or more atoms or radicals selected from halogen atoms and C₁-C₄ alkoxy, mono- or polyhalo(C₁-C₄ alkoxy), C₂-C₄ alkenyl, C₂-C₄ alkynyl, mono- or polyhalo(C₂-C₄ alkenyl) and mono- or polyhalo(C₂-C₄ alkynyl) radicals), or C₃-C₇ cycloalkyl, C₆-C₁₀ aryl or C₇-C₁₁ aralkyl radicals, it being possible for these various radicals to be optionally substituted (with one or more atoms or radicals selected from halogen atoms and C₁-C₄ alkyl radicals, mono- or polyhalo(C₁-C₄ alkyl) radicals, C₁-C₄ alkoxy radicals and mono- or polyhalo(C₁-C₄ alkoxy) radicals), or a C₂-C₄ ethynyl, C₂-C₄ acetynyl, mono- or polyhalo(C₂-C₄ ethynyl) or mono- or polyhalo(C₂-C₄ acetynyl) radical,
R₁₂ has one of the meanings of R₅, with the exception of C(=O)-R₁₃, and
R₄ denotes a hydrogen atom, a halogen atom, in particular a chlorine or bromine atom, a C₁-C₄ alkyl radical, optionally substituted (with one or more atoms or radicals selected from halogen atoms and C₁-C₄ alkoxy, mono- or polyhalo(C₁-C₄ alkoxy), C₂-C₄ alkenyl, C₂-C₄ alkynyl, mono- or polyhalo(C₂-C₄ alkenyl) and mono- or polyhalo(C₂-C₄ alkynyl) radicals), or C₃-C₇ cycloalkyl, C₆-C₁₀ aryl or C₇-C₁₁ aralkyl radicals, it being possible for these various radicals to be optionally substituted (with one or more atoms or radicals selected from halogen atoms and C₁-C₄ alkyl radicals, mono- or polyhalo(C₁-C₄ alkyl) radicals, C₁-C₄ alkoxy radicals and mono- or polyhalo (C₁-C₄ alkoxy) radicals), and the agriculturally acceptable salified forms of these compounds.

2. Compounds according to claim 1, characterized in that n = 1, 2 or 3.

3. Compounds according to claim 2, characterized in that X is a halogen atom selected from chlorine, bromine or fluorine.

4. Compounds according to claim 3, characterized in that n = 1 or 2 and X is located at the para-position when n = 1 and at the meta- and para- or ortho- and para-positions when n = 2.

5. Compounds according to claim 4, characterized in that n = 1.

6. Compounds according to claim 3, 4 or 5, characterized in that X is a chlorine atom.

7. Compounds according to claim 1, characterized in that W is a nitrogen atom.

8. Compounds according to claim 1, characterized in that R₃, R₆, R₉ and R₁₀ are a hydrogen atom.

9. Compounds according to claim 8, characterized in that R₄, R₇, R₉ and R₁₁ are a hydrogen atom or a C₁-C₄ alkyl radical.

10. Compounds according to claim 1, characterized in that R₁ and R₂ are selected from methyl or ethyl radicals or a hydrogen atom.

11. Compounds according to claim 1, characterized in that R₅ is a hydrogen atom.

12. Compounds according to claim 1, characterized in that R₁₂ is a hydrogen atom.

13. Compounds according to claim 1, characterized in that they are of formula IA.

14. Compounds according to claim 13, characterized in that, when A is CR₆R₇, R₁ and R₂ are selected from methyl or ethyl radicals, R₃, R₅ to R₇ and R₁₂ are a hydrogen atom and R₄ is methyl, ethyl, isopropyl, n-propyl or a hydrogen atom.

15. Compounds according to claim 14, which are 2-(4-chlorobenzylidene)-5,5-dimethyl-1-(1H-1,2,4-triazol-1-ylmethyl)-1-cyclopentanol or 2-(4-chlorobenzylidene)-5-methyl-5-ethyl-1-(1H-1,2,4-triazol-1-ylmethyl)-1-cyclopentanol.

16. Compounds according to claim 13, characterized in that, when A is CR₆R₇CR₈R₉, R₁ and R₂ are selected from methyl and ethyl radicals or a hydrogen atom, R₃, R₅ to R₉ and R₁₂ are a hydrogen atom and R₄ is a methyl, ethyl, isopropyl or n-propyl radical or a hydrogen atom.

17. Compounds according to claim 13, which are
- 2-(4-chlorobenzylidene)-6-methyl-1-(1H-1,2,4-triazol-1-ylmethyl)-1-cyclohexanol,
- 2-(4-chlorobenzylidene)-6,6-dimethyl-1-(1H-1,2,4-triazol-1-ylmethyl) or
- 2-(4-chlorobenzylidene)-1-(1H-1,2,4-triazol-1-ylmethyl)-1-cyclohexanol.

18. Fungicidal composition comprising 0.5 % to 95 % of compounds according to one of claims 1 to 17, in combination with agriculturally acceptable solid or liquid vehicles and/or these surfactant agents which are also agriculturally acceptable.

19. Process for treating crops attacked or liable to be attacked by fungal diseases, characterized in that an effective amount of a compound according to one of claims 1 to 17 or a composition according to claim 18 is applied on the leaves.

20. Treatment process according to claim 19, characterized in that a dose of between 0.002 and 5 kg/ha is applied.

21. Product of multiplication of cultivated plants which has undergone a protective treatment with one of the compounds according to one of claims 1 to 17 or a composition according to claim 18.

22. Grain according to claim 21, which has undergone a protective treatment at the rate of 0.1 to 500 g per quintal.

23. Compounds which are useful, in particular, as intermediates in the preparation of the compounds according to claims 1 to 17, corresponding to the formulae: 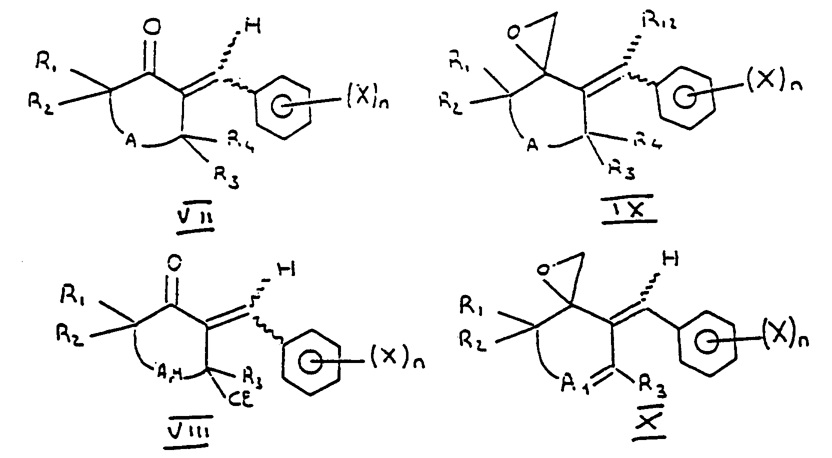 in which:
- the substituents n, X, R₁, R₂, A₁, R₄, A, R₃ and R₁₂ have the same meaning as in claim 1,
- and the group A₁H of the formula VIII corresponds to the groups -CR₇H, -CR₆R₇-CR₉H or - -CR₆R₇-CR₈R₉-CR₁₁H, in which the substituents R₆, R₇, R₈, R₉ and R₁₁ have the same meaning as in claim 1,
except for the following compounds:
2-benzylidenecyclopentanone,
2-(4-chlorobenzylidene)cyclopentanone,
2-(4-chlorobenzylidene)-5-benzylcyclopentanone,
2-(4-bromobenzylidene)-5-benzylcyclopentanone,
2-benzylidene-3-methylcyclopentanone,
2-benzylidene-5-methylcyclopentanone,
2-benzylidene-5-benzylcyclopentanone,
2-benzylidenecyclohexanone,
6-cyclohexyl-2-benzylidenecyclohexanone,
2-benzylidene-5-methyl-6-cyclohexylcyclohexanone,
2-(4-methoxybenzylidene)-4-methyl-6-phenylcyclohexanone,
2-benzylidene-6(4-isopropylphenyl)cyclohexanone,
2-benzylidene-6-methylcyclohexanone,
2-benzylidene-4-methylcyclohexanone,
2-benzylidenecycloheptanone,
2-benzylidene-6-benzylcyclohexanone; and with the exclusion of the compounds of formula VII, in which:
- R₁ is a hydrogen atom; and
- R₂ is a hydrogen atom or a cyclohexyl or phenyl radical, the said radical being optionally substituted with a C₁-C₄ alkyl radical; and
- R₃ is a hydrogen atom; and
- R₄ is a hydrogen atom or a methyl, ethyl or propyl radical; and
- A is -CHR₆-, -CHR₆-CHR₈- or -CHR₆-CHR₈-CHR₁₀-; and
- R₆, R₈ and R₁₀ are selected from a hydrogen atom and a methyl, ethyl or propyl radical; and
- n is equal to 0, 1 or 2; and
- X is a hydrogen or halogen atom, a C₁-C₄ alkyl or C₁-C₄ alkoxy radical, or a nitro group.

24. Process for preparing compounds of formula IA in which R₁, R₂, R₃, R₁₂, X, n, W and A have the same meaning as in claim 1, R₄ is other than a halogen atom and R₅ is a hydrogen atom, characterized in that a compound of formula VII is reacted with a sulphonium ylide to lead to the oxiranes of formula IX, and the latter is then reacted with an unsubstituted imidazole or triazole in the presence of a base.

25. Process for preparing compounds of formula IA in which R₁, R₂, R₃, R₁₂, X, n, W and A have the same meaning as in claim 1, R₄ is a halogen atom and R₅ is a hydrogen atom, by allyl halogenation of the compounds of formula (I) in which R₄ and R₅ are a hydrogen atom.

26. Process for preparing compounds of formula IA in which R₁, R₂, R₃, R₁₂, X, n, W and A have the same meaning as in claim 1, R₄ is other than a halogen atom and R₅ is other than a hydrogen atom, by esterification or etherification of the compounds of formula IA obtained according to claim 24.

27. Process for preparing compounds of formula IA in which R₁, R₂, R₃, R₁₂, X, n, W and A have the same meaning as in claim 1, R₄ is a halogen atom and R₅ is other than a hydrogen atom, are obtained, in a first step, by esterification or etherification, according to claim 26, of a compound of formula I in which R₄ and R₅ are a hydrogen atom, followed by an allyl halogenation.

28. Process for preparing compounds of formula IB in which R₁, R₂, A, n and X have the same meaning as in claim 1 and R₅ is a halogen atom, characterized in that a compound of formula VIII is reacted with a sulphonium ylide to obtain an oxirane of formula X, followed by a reaction with an imidazole or triazole in the presence of a base, optionally followed by an esterification or etherification to obtain a compound in which R₅ is a hydrogen atom.

## Patentansprüche

1. Benzylidenazolylmethylcycloalkane oder -alkene der Formeln 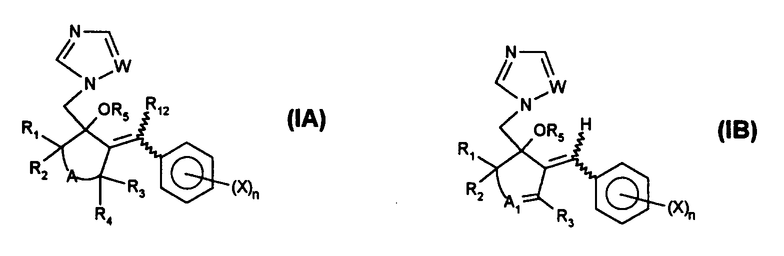 in denen
A -CR₆ R₇- oder CR₆ R₇ CR₈ R₉- oder -CR₆ R₇ CR₈ R₉ CR₁₀ R₁₁- ist;
A1 CR₇=, -CR₆ R₇-CR₉= oder -CR₆ R₇-CR₈ R₉-CR₁₁= ist;
X eine Cyano- oder Nitrogruppe oder eine C₁-C₄ Alkyl- oder C₁-C₄ - Alkoxygruppe, die gegebenenfalls halogeniert sind, ist;
n eine ganze positive Zahl, die kleiner als 6 ist, oder Null ist, wobei die Reste X identisch oder verschieden sein können, wenn n größer als 1 ist;
W eine trivalente Gruppe darstellt, die entweder aus einer =CH-Gruppe oder aus einem Stickstoffatom =N-, besteht
R₁, R₂ identisch oder verschieden sind und ein Wasserstoffatom darstellen oder einen C₁-C₄-Alkylrest, der gegebenenfalls substituiert ist (z.B. mit einem oder mehreren Atomen oder Resten, ausgewählt aus Halogenatomen, C₁-C₄-Alkoxyresten, mono- oder polyhalogenierten C₁-C₄-Alkoxyresten, C₂-C₄-Alkenylresten, C₂-C₄-Alkinylresten, mono- oder polyhalogenierten C₂-C₄-Alkenylresten, mono- oder polyhalogenierten C₂-C₄-Alkinylresten), einen C₁-C₄-Alkoxyrest, der gegebenenfalls substituiert ist (z.B. mit einem oder mehreren Atomen oder Resten, ausgewählt aus Halogenatomen, C₁-C₄-Alkoxyresten, mono- oder polyhalogenierten C₁-C₄-Alkoxyresten, C₂-C₄-Alkenylresten, C₂-C₄-Alkinylresten, mono- oder polyhalogenierten C₂-C₄-Alkenylresten, mono- oder polyhalogenierten C₂-C₄-Alkinylresten) oder einen C₃-C₇-Cycloalkylrest, einen C₆-C₁₀-Arylrest, einen C₇-C₁₁-Aralkylrest, wobei diese verschiedenen Reste gegebenenfalls substituiert sein können (z.B. mit einem oder mehreren Atomen oder Resten, ausgewählt aus Halogenatomen, C₁-C₄-Alkylresten, mono- oder polyhalogenierten C₁-C₄-Alkylresten, C₁-C₄-Alkoxyresten und mono- oder polyhalogenierten C₁-C₄-Alkoxyresten), wobei R₁ und R₂ zusammen eine C₂-C₅-Kohlenwasserstoffkette bilden können, die einen Ring bildet mit dem Kohlenstoff, an den R₁ und R₂ gebunden sind, wobei diese Kette gegebenenfalls substituiert ist, wie bei den vorgenannten C₆-C₁₀-Arylresten angegeben, oder wobei R₁ und R₂ zusammen eine C₂-C₅-Kohlenwasserstoff-Dioxolankette bilden können mit dem Kohlenstoff an den R₁ und R₂ gebunden sind, wobei diese Kette gegebenenfalls substituiert sein kann wie bei den vorgenannten C₆-C₁₀-Arylresten beschrieben;
R₃, R₆ bis R₁₁ identisch oder verschieden sind und jeweils ein Wasserstoffatom oder einen C₁-C₄-Alkylrest darstellen, der gegebenenfalls substituiert ist (z.B. mit einem oder mehreren Atomen oder Resten, ausgewählt aus Halogenatomen, C₁-C₄-Alkoxyresten, mono- oder polyhalogenierten C₁-C₄-Alkoxyresten), oder einen C₃-C₇-Cycloalkylrest oder einen C₆-C₁₀ Arylrest oder einen C₇-C₁₁-Aralkylrest, wobei diese verschiedenen Reste gegebenenfalls substituiert sein können (z.B. mit einem oder mehreren Atomen oder Resten, ausgewählt aus Halogenatomen, C₁-C₄-Alkylresten, mono- oder polyhalogenierten C₁-C₄-Alkylresten, C₁-C₄-Alkoxyresten und mono- oder polyhalogenierten C₁-C₄-Alkoxyresten), oder wobei zwei benachbarte Reste der Kette A zusammen mit den Atomen von A, an die sie gebunden sind, einen kondensierten Phenylring oder Cycloalkanring bilden;
R₅ ein Wasserstoffatom darstellt, einen C₁-C₄-Alkylrest, der gegebenenfalls substituiert ist (z.B. mit einem oder mehreren Atomen oder Resten, ausgewählt aus Halogenatomen, C₁-C₄-Alkoxyresten, mono- oder polyhalogenierten C₁-C₄-Alkoxyresten, C₂-C₄-Alkenylresten, C₂-C₄-Alkinylresten, mono- oder polyhalogenierten C₂-C₄-Alkenylresten, mono- oder polyhalogenierten C₂-C₄-Alkinylresten), einen C₃-C₇-Cycloalkylrest, einen C₆-C₁₀-Arylrest oder einen C₇-C₁₁-Aralkylrest, wobei diese verschiedenen Reste gegebenenfalls substituiert sein können (z.B. mit einem oder mehreren Atomen oder Resten, ausgewählt aus Halogenatomen, C₁-C₄-Alkylresten, mono- oder polyhalogenierten C₁-C₄-Alkylresten, C₁-C₄-Alkoxyresten und mono- oder polyhalogenierten C₁-C₄-Alkoxyresten), oder R₅ einen Rest C(=O)-R₁₃ darstellt, wobei R₁₃ einen C₁-C₄-Alkylrest darstellt, der gegebenenfalls substituiert ist (z.B. mit einem oder mehreren Atomen oder Resten, ausgewählt aus Halogenatomen, C₁-C₄-Alkoxyresten, mono- oder polyhalogenierten C₁-C₄-Alkoxyresten, C₂-C₄-Alkenylresten, C₂-C₄-Alkinylresten, mono- oder polyhalogenierten C₂-C₄-Alkenylresten, mono oder polyhalogenierten C₂-C₄-Alkinylresten), einen C₃-C₇ Cyloalkylrest, einen C₆-C₁₀-Arylrest oder einen C₇-C₁₁-Aralkylrest, wobei diese verschiedenen Reste gegebenenfalls substituiert sind (z.B. mit einem oder mehreren Atomen oder Resten, ausgewählt aus Halogenatomen, C₁-C₄-Alkylresten, mono- oder polyhalogenierten C₁-C₄-Alkylresten, C₁-C₄-Alkoxyresten und mono-oder polyhalogenierten C₁-C₄-Alkoxyresten) oder einen C₂-C₄-Ethinylrest, einen C₂-C₄ Acetinylrest, einen mono- oder polyhalogenierten C₂-C₄-Ethinylrest oder mono- oder polyhalogenierten C₂-C₄-Acetinylrest;
R₁₂ eine der für R₅ angegebenen Bedeutungen hat mit Ausnahme von C(=O)-R₁₃;
R₄ ein Wasserstoffatom, ein Halogenatom, insbesondere ein Chloratom oder Bromatom darstellt, einen C₁-C₄-Alkylrest, der gegebenenfalls substituiert ist (z.B. mit einem oder mehreren Atomen oder Resten, ausgewählt aus Halogenatomen, C₁-C₄-Alkoxyresten, mono- oder polyhalogenierten C₁-C₄-Alkoxyresten, C₂-C₄-Alkenylresten, C₂-C₄-Alkinylresten, mono- oder polyhalogenierten C₂-C₄-Alkenylresten, mono- oder polyhalogenierten C₂-C₄-Alkinylresten), einen C₃-C₇-Cycloalkylrest, einen C₆-C₁₀-Arylrest oder einen C₇-C₁₁-Aralkylrest, wobei diese verschiedenen Reste gegebenenfalls substituiert sein können (z.B. mit einem oder mehreren Atomen oder Resten, ausgewählt aus Halogenatomen, C₁-C₄-Alkylresten, mono- oder polyhalogenierten C₁-C₄-Alkylresten, C₁-C₄-Alkoxyresten und mono- oder polyhalogenierten C₁-C₄-Alkoxyresten), sowie die in der Landwirtschaft verträglichen Salzformen dieser Verbindungen.

2. Verbindungen nach Anspruch 1, dadurch gekennzeichnet, daß n = 1, 2 oder 3 ist.

3. Verbindungen nach Anspruch 2, dadurch gekennzeichnet, daß X ein Halogenatom ist, ausgewählt aus Chlor, Brom und Fluor.

4. Verbindungen nach Anspruch 3, dadurch gekennzeichnet, daß n = 1 oder 2 ist und X in para-Position steht, wenn n = 1 ist, und in meta-para- oder ortho-para-Position, wenn n = 2 ist.

5. Verbindungen nach Anspruch 4, dadurch gekennzeichnet, daß n = 1 ist.

6. Verbindungen nach Anspruch 3, 4 oder 5, dadurch gekennzeichnet, daß X ein Chloratom ist.

7. Verbindungen nach Anspruch 1, dadurch gekennzeichnet, daß W ein Stickstoffatom ist.

8. Verbindungen nach Anspruch 1, dadurch gekennzeichnet, daß R₃, R₆, R₉ und R₁₀ Wasserstoffatome darstellen.

9. Verbindungen nach Anspruch 8, dadurch gekennzeichnet, daß R₄, R₇, R₉ und R₁₁ ein Wasserstoffatom oder einen C₁-C₄-Alkylrest darstellen.

10. Verbindungen nach Anspruch 1, dadurch gekennzeichnet, daß R₁ und R₂ ausgewählt sind aus den Resten Methyl oder Ethyl oder ein Wasserstoffatom darstellen.

11. Verbindungen nach Anspruch 1, dadurch gekennzeichnet, daß R₅ ein Wasserstoffatom ist.

12. Verbindungen nach Anspruch 1, dadurch gekennzeichnet, daß R₁₂ ein Wasserstoffatom ist.

13. Verbindungen nach Anspruch 1, dadurch gekennzeichnet, daß sie der Formel IA entsprechen.

14. Verbindungen nach Anspruch 13, dadurch gekennzeichnet, daß, wenn A CR₆R₇ ist, R₁ und R₂ ausgewählt sind aus den Resten Methyl oder Ethyl, R₃ und R₅ bis R₇ und R₁₂ ein Wasserstoffatom darstellen und R₄ Methyl, Ethyl, i-Propyl, n-Propyl oder ein Wasserstoffatom darstellt.

15. Verbindungen nach Anspruch 14, die 2-(4-Chlorbenzyliden)-5,5-dimethyl-1-(1H-1,2,4-triazol-1-ylmethyl)-1-cyclopentanol ist oder 2-(4-Chlorbenzyliden)-5-methyl-5-ethyl-1-(1H-1,2,4-triazol-1-ylmethyl)-1-cylopentanol sind.

16. Verbindungen nach Anspruch 13, dadurch gekennzeichnet, daß, wenn A CR₆R₇ CR₈R₉ ist, R₁ und R₂ ausgewählt sind aus den Resten Methyl, Ethyl oder ein Wasserstoffatom sind, R₃ und R₅ bis R₉ und R₁₂ ein Wasserstoffatom sind und R₄ ein Methylrest, Ethylrest, i-Propylrest, n-Propylrest oder ein Wasserstoffatom ist.

17. Verbindungen nach Anspruch 13, die 2-(4-Chlorbenzyliden)-6-methyl-1-(1H-1,2,4-triazol-1-ylmethyl)-1-cyclohexanol oder 2-(4-Chlorbenzyliden)-6,6-dimethyl-1-(1H-1,2,4-triazol-1-ylmethyl)-1-cyclohexanol oder 2-(4-Chlorbenzyliden)-1-(1H-1,2,4-triazol-1-ylmethyl)-1-cyclohexanol sind.

18. Fungizide Zusammensetzung, enthaltend 0,5 % bis 95 % der Verbindungen nach einem der Ansprüche 1 bis 17 zusammen mit festen oder flüssigen, für die Landwirtschaft vertraglichen Trägern und/oder oberflächenaktiven, ebenfalls für die Landwirtschaft vertraglichen Stoffen.

19. Verfahren zur Behandlung von Kulturen, die mit Pilzkrankheiten befallen sind oder denen Befall droht, dadurch gekennzeichnet, daß man auf die Blätter eine wirksame Menge einer Verbindung nach einem der Ansprüche 1 bis 17 oder eine Zusammensetzung nach Anspruch 18 aufbringt.

20. Verfahren zur Behandlung nach Anspruch 19, dadurch gekennzeichnet, daß man eine Menge zwischen 0,002 und 5 kg/ha verwendet.

21. Produkt zur Vermehrung von Kulturpflanzen, das einer Schutzbehandlung mit einer der Verbindungen nach einem der Ansprüche 1 bis 17 oder einer Zusammensetzung nach Anspruch 18 unterworfen wurde.

22. Samenkorn nach Anspruch 21, das einer Schutzbehandlung mit einer Menge von 0,1 bis 500 g/Doppelzentner unterzogen wurde.

23. Verbindungen, die insbesondere als Zwischenprodukt bei der Herstellung von Verbindungen nach den Ansprüchen 1 bis 17 verwendbar sind und den Formeln 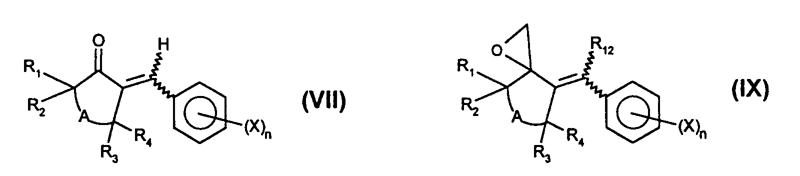 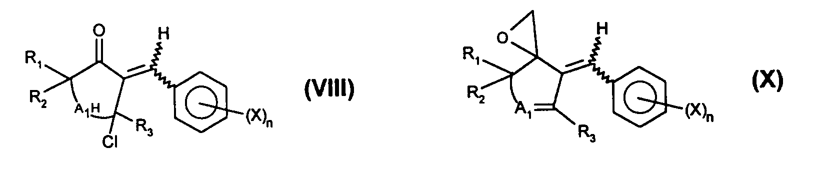 entsprechen, in denen
- die Substituenten n, X, R₁, R₂, A₁, A, R₃, R₄, R₁₂ dieselbe Bedeutung haben wie in Anspruch 1,
- und die Gruppe A₁H der Formel VIII den Gruppen -CR₇H oder -CR₆R₇-CR₉H oder -CR₆R₇-CR₈R₉-CR₁₁H entspricht, wobei die Substituenten R₆, R₇, R₈, R₉, R₁₁ dieselbe Bedeutung wie in Anspruch 1 haben,
außer den folgenden Verbindungen:
2-Benzylidencyclopentanon,
2-(4-Chlorbenzyliden)-cyclopentanon,
2-(4-Chlorbenzyliden)-5-benzylcyclopentanon,
2-(4-Brombenzyliden)-5-benzylcyclopentanon,
2-Benzyliden-3-methylcyclopentanon,
2-Benzyliden-5-methylcyclopentanon,
2-Benzyliden-5-benzylcyclopentanon,
2-Benzylidencyclohexanon,
6-Cyclohexyl-2-benzylidencyclohexanon,
2-Benzyliden-5-methyl-6-cyclohexylcyclohexanon,
2-(4-Methoxybenzyliden)-4-methyl-6-phenylcyclohexanon,
2-Benzyliden-6-(4-isopropylphenyl)-cyclohexanon,
2-Benzyliden-6-methylcyclohexanon,
2-Benzyliden-4-methylcyclohexanon,
2-Benzylidencycloheptanon,
2-Benzyliden-6-benzylcyclohexanon;
und mit Ausnahme der Verbindungen der Formel VII, in denen
- R₁ ein Wasserstoffatom ist; und
- R₂ ein Wasserstoffatom oder ein Cyclohexyl- oder Phenylrest ist, wobei dieser Rest gegebenenfalls mit einem C₁-C₄-Alkylrest substituiert ist; und
- R₃ ein Wasserstoffatom ist; und
- R₄ ein Wasserstoffatom oder ein Methyl-, Ethyl- oder Propylrest ist; und
- A -CHR₆-, -CHR₆-CHR₈-, -CHR₆-CHR₈-CHR₁₀- ist; und
- R₆, R₈, R₁₀ ausgewählt sind aus einem Wasserstoffatom oder einem Methyl-, Ethyl- oder Propylrest; und
- n 0, 1 oder 2 ist; und
- X ein Wasserstoffatom oder ein Halogenatom oder ein C₁-C₄-Alkylrest oder ein C₁-C₄-Alkoxyrest oder eine Nitrogruppe ist.

24. Verfahren zur Herstellung von Verbindungen der Formel IA in der R₁, R₂, R₃, R₁₂, X, n, W und A dieselbe Bedeutung haben wie in Anspruch 1, R₄ von einem Wasserstoffatom verschieden ist, R₅ ein Wasserstoffatom ist, dadurch gekennzeichnet, daß eine Verbindung der Formel VII mit einem Sulfoniumylid zur Reaktion gebracht wird, um Oxirane der Formel IX zu erhalten, und dann dieses mit einem nichtsubstituierten Imidazol oder Triazol in Gegenwart einer Base zur Reaktion gebracht wird.

25. Verfahren zur Herstellung von Verbindungen der Formel IA, in der R₁, R₂, R₃, R₁₂, X, n, W und A dieselbe Bedeutung haben wie in Anspruch 1, R₄ ein Halogenatom ist, R₅ ein Wasserstoffatom ist, durch allylische Halogenierung der Verbindungen der Formel (I), in denen R₄ und R₅ ein Wasserstoffatom sind.

26. Verfahren zur Herstellung von Verbindungen der Formel IA, in der R₁, R₂, R₃, R₁₂, X, n, W und A dieselbe Bedeutung haben wie in Anspruch 1, R₄ von einem Halogenatom verschieden ist und R₅ von einem Wasserstoffatom verschieden ist, durch Veresterung oder Veretherung von Verbindungen der Formel IA, die nach Anspruch 24 erhalten wurden.

27. Verfahren zur Herstellung von Verbindungen der Formel IA, in der R₁, R₂, R₃, R₁₂, X, n, W und A dieselbe Bedeutung haben wie in Anspruch 1, R₄ ein Halogenatom ist, R₅ von einem Wasserstoffatom verschieden ist, die erhalten wurden in einem ersten Schritt durch Veresterung oder Veretherung nach Anspruch 26 einer Verbindung der Formel I, in der R₄ und R₅ ein Wasserstoffatom darstellen, worauf eine allylische Halogenierung folgt.

28. Verfahren zur Herstellung von Verbindungen der Formel IB, in der R₁, R₂, A, n, X dieselbe Bedeutung haben wie in Anspruch 1, R₅ ein Halogenatom ist, dadurch gekennzeichnet, daß man eine Verbindung der Formel VIII mit einem Sulfoniumylid reagieren läßt, um ein Oxiran der Formel X zu erhalten, worauf eine Reaktion mit einem Imidazol oder Triazol in Gegenwart einer Base folgt, worauf gegebenenfalls eine Veresterung oder Veretherung erfolgt, um eine Verbindung zu erhalten, in der R₅ ein Wasserstoffatom ist.
